(19) Europäisches Patentamt
European Patent Office
Office européen des brevets

(11) EP 4 203 914 B1

(12) EUROPEAN PATENT SPECIFICATION

(45) Date of publication and mention of the grant of the patent:
**07.05.2025 Bulletin 2025/19**

(21) Application number: **21836722.5**

(22) Date of filing: **09.12.2021**

(51) International Patent Classification (IPC):
***A61K 8/67*** *(2006.01)* ***A61K 8/44*** *(2006.01)*
***A61K 8/36*** *(2006.01)* ***A61Q 11/00*** *(2006.01)*

(52) Cooperative Patent Classification (CPC):
**A61K 8/676; A61K 8/361; A61K 8/442; A61Q 11/00**

(86) International application number:
**PCT/US2021/062627**

(87) International publication number:
**WO 2022/125786 (16.06.2022 Gazette 2022/24)**

# (54) ORAL CARE COMPOSITIONS COMPRISING ASCORBIC ACID DERIVATIVES

MUNDPFLEGEZUSAMMENSETZUNGEN MIT ASCORBINSÄUREDERIVATEN

COMPOSITIONS DE SOINS BUCCAUXDENTAIRES COMPRENANT DES DÉRIVÉS D’ACIDE ASCORBIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2020 US 202063123016 P**

(43) Date of publication of application:
**05.07.2023 Bulletin 2023/27**

(73) Proprietor: **Colgate-Palmolive Company**
**New York, NY 10022 (US)**

(72) Inventors:
- **NORTH, Michael**
  **Middlesex, New Jersey 08846 (US)**
- **CARROLL, Keith**
  **Robbinsville, New Jersey 08691 (US)**
- **GRONLUND, Jennifer**
  **Flemington, New Jersey 08822 (US)**

(74) Representative: **Sonnenhauser, Thomas Martin**
**Wuesthoff & Wuesthoff**
**Patentanwälte und Rechtsanwalt PartG mbB**
**Schweigerstraße 2**
**81541 München (DE)**

(56) References cited:
**WO-A1-2004/041228**
**WO-A1-2010/074025**
**JP-A- 2005 075 825**
**JP-A- 2005 239 654**
**JP-A- 2005 320 321**
**JP-A- 2007 297 295**
**JP-A- 2007 308 443**
**JP-A- H1 143 424**
**JP-A- H10 245 328**
**US-A1- 2004 175 332**
**US-A1- 2008 057 007**
**US-A1- 2008 112 903**
**US-A1- 2019 076 496**
**US-A1- 2019 185 499**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 203 914 B1

## Description

### FIELD OF THE DISCLOSURE

In one aspect the disclosure relates to oral care compositions that make teeth whiter. In one aspect, the oral care compositions described herein comprise ascorbic acid derivatives as teeth whitening agents. In another aspect, the oral care compositions described herein are stable until use, reasonably priced, safe, easy to use, and do not require special apparatus or trained personnel to be applied.

### BACKGROUND OF THE DISCLOSURE

Foods, tobacco, medication, drinks such as coffee, tea and red wine, stain teeth overtime. Whitening teeth can be achieved by using tooth whitening products. Oral care compositions and processes have been developed over the years to whiten teeth. Some of the most common oral care compositions for whitening teeth contain peroxide derivatives. Peroxide compounds can bleach teeth, remove stains and kill cariogenic bacteria. However, the lack of compound stability, the cost, the harshness to teeth and gums represents downsides of the available processes. Consumers are turning more and more towards "natural" ingredients within their oral care products. By "natural" ingredients it is to be understood as "naturally sourced" and "naturally derived" ingredients.

Therefore, there is a clear need for natural oral care compositions for teeth whitening, which are stable until use, reasonably priced, safe, easy to use, and which do not require special apparatus or trained personnel to be applied. WO 2004/041228 A1 discloses the use of a polymer for improving the uptake and retention of water-soluble ascorbic acid derivatives from oral care compositions onto the surface of teeth and the surrounding dental tissue. US 2008/0112903 A1 discloses topical oral care compositions including a free radical scavenging precursor compound. US 2019/0185499 A1 discloses stannous-ascorbyl phosphate and stannic-ascorbyl phosphate complexes, oral care compositions comprising stannous-ascorbyl phosphate or stannic-asorbyl phosphate complexes, and methods of making and using the same. JP 2007-308443 A discloses a cataplasm product for oral cavity comprising a paste composition containing (A) one or more kinds of compounds selected from an ascorbate and its salt, (B) a gelling agent, (C) water and/or organic solvent having 0-7,000 kPa vapor pressure (at 25°C) in an amount of 30.0-99.0 mass% in the whole composition and (D) an alkali agent, and having a pH of 8.0-9.5 and a support for attaching the paste composition to at least one part selected from teeth, oral mucosa and gums. US 2004/175332 A1 discloses a method for suppressing discoloration of a composition in which a discoloration suppressing amount of ethylenediaminetetraacetic acid or a salt thereof is incorporated into a composition containing an ascorbic acid derivative. WO 2010/074025 A1 discloses a dentifrice composition characterized by containing (A) at least one or more selected from ascorbic acid ester and salts thereof and (B) aluminum lactate and/or potassium nitrate, wherein the content ratio of ingredient (B)/ingredient (A) is 2-50 by mass ratio.

### SUMMARY OF THE DISCLOSURE

The present disclosure relates to oral care compositions for whitening teeth as defined in the claims. The oral care compositions of the disclosure comprise reducing agents as teeth whitening agents as defined in the claims. Traditional whitening agents are believed to act as oxidizing agents (accepting electrons) to bleach stains. Without being bound by theory, ascorbic acid and its derivatives are able to bleach stains by acting as reducing agents (losing electrons). In some aspects, the oral care compositions described herein comprise whitening agents in a buffered oral care composition providing protection from tooth erosion. Claimed is an oral care composition for whitening dental enamel, wherein the oral care composition comprises an effective amount of an ascorbic acid derivative, wherein the ascorbic acid derivative is sodium ascorbyl phosphate; wherein the ascorbic acid derivative is present in an amount of about 1.5% by wt. of the total composition; and a zwitterionic surfactant as defined in the claims. Further claimed is a non-therapeutic method for whitening a surface of a tooth as defined in the claims. Further claimed is a method for increasing the color stability of an oral care composition comprising an ascorbic acid derivative as defined in the claims. Further claimed is a non-therapeutic method to treat or reduce staining of the enamel as defined in the claims.

In one aspect, the oral care compositions of the disclosure do not comprise bleaching agents or whitening agents such as hydrogen peroxide or a hydrogen peroxide source, for example, urea peroxide, or metal chlorites or a peroxide salt or complex (for example, peroxyphosphate, peroxycarbonate, perborate, peroxysilicate), or persulfate salts (for example calcium peroxyphosphate, sodium perborate, sodium carbonate peroxide, sodium peroxyphosphate, and potassium persulfate), or a hydrogen peroxide polymer complex (for example, a peroxide-polyvinyl pyrrolidone polymer complex).

The oral care compositions of the disclosure comprise ascorbic acid derivatives as defined in the claims as whitening agents. Ascorbic acid is a natural water-soluble vitamin, commonly known as vitamin C. Ascorbic acid exists as two enantiomers commonly denoted "l" (for "levo") and "d" (for "dextro"). The "l" isomer is the one most often encountered. Ascorbic acid is also referred to as L (+)-ascorbic acid or l-ascorbic acid.

The oral care composition of the disclosure comprises an ascorbic acid derivative that is sodium ascorbyl phosphate as defined in the claims. Generally, derivatives can be selected from the group consisting of: L-ascorbic acid, calcium ascorbate, calcium I-ascorbate dihydrate, magnesium ascorbate, potassium ascorbate, magnesium L-ascorbyl phosphate (also referred to as: magnesium ascorbate phosphate or ascorbic acid phosphate magnesium salt), L-ascorbic acid 2-phosphate sesquimagnesium salt hydrate, (+) sodium L-ascorbate, dehydro-I-(+)-ascorbic acid dimer, sodium ascorbyl phosphate (also referred to as: ascorbic acid phosphate sodium salt, sodium I-ascorbyl phosphate, 2-phospho-L-ascorbic acid trisodium salt, L-ascorbic acid 2-phosphate trisodium salt or sodium L-ascorbyl-2-phosphate), ascorbic acid-2-glucoside, ascorbyl dipalmitate, ascorbyl methylsilanol pectinate, ascorbyl stearate, disodium ascorbyl sulfate, ascorbyl 6-palmitate and combinations thereof.

In one embodiment, the oral care composition of the disclosure is in the form selected from: a toothpaste, a dentifrice, a mouthwash, a mouthrinse, a topical oral gel and a denture cleanser. In another embodiment, the oral care composition of the disclosure is in the form selected from: dental strips, beads, ribbon toothpaste, varnish, dental floss, chewing gum, lozenges and toothpowder.

In yet a further aspect, the oral care compositions of the disclosure can comprise antiplaque agents, antigingivitis agents, antimalodor agents, antitartar agents, anticalculus agents, erosion prevention agents, and/or combinations thereof. Optionally, the oral care compositions of the disclosure may comprise abrasives, surfactants, viscosity and rheology modifiers, humectants, sweeteners, flavors, colorants, preservatives and/or combinations thereof. Optionally, the oral care composition of the disclosure comprises water. Optionally, the oral care compositions of the disclosure may comprise pigments, opacifiers, polymer complexes, surfactants, bicarbonate salts, pH modifying agents, foam modulators, thickening agents, antibacterial agents, anticariogenic agents, fluoride ion sources, stannous ion sources, zinc ion sources and/or combinations thereof.

In one aspect, the compositions of the disclosure contain a relatively high amount of sodium stearate. Without being bound by theory, the relatively high amounts of sodium stearate can help with the toothpaste discoloration that can take place with the presence of ascorbic acid derivatives (e.g., sodium ascorbyl phosphate or sodium and ascorbyl 6-palmitate).

The compositions of the disclosure can be utilized in methods of removing surface enamel stains (e.g., not intrinsic stain removal). In one aspect, without being bound by theory, the phosphate salt on the ascorbic acid derivative can help lift the stain from the surface of the enamel while the fatty acid chain can help anchor the derivative to surface in order to perform this task.

## DETAILED DESCRIPTION

It is to be understood that both the foregoing general description and the following detailed description of the disclosure, are exemplary and explanatory only and are not restrictive of the claimed disclosure which is defined in the claims.

As used herein, the use of the singular includes the plural unless specifically stated otherwise. As used throughout, ranges are used as shorthand for describing each and every value that is within the range. As used herein, the use of a compound comprising several isomers or stereoisomers, includes all the isomeric forms of that compound.

Unless stated otherwise, all percentages of the oral care composition components given in this specification are by weight based on a total oral care composition or formulation weight of 100%.

As used herein, an "oral care composition" refers to a composition for which the intended use includes oral care, oral hygiene, and/or oral appearance, or for which the intended method of use comprises administration to the oral cavity, and refers to compositions that are palatable and safe for topical administration to the oral cavity, and for providing a benefit to the teeth and/or oral cavity. The term "oral care composition" thus specifically excludes compositions which are highly toxic, unpalatable, or otherwise unsuitable for administration to the oral cavity. In some embodiments, an oral care composition is not intentionally swallowed, but is rather retained in the oral cavity for a time sufficient to affect the intended utility. The oral care compositions as disclosed herein may be used in nonhuman mammals such as companion animals (e.g., dogs and cats), as well as by humans. In some embodiments, the oral care compositions as disclosed herein are used by humans. Oral care compositions include, for example, dentifrice and mouthwash. In some embodiments, the disclosure provides mouthwash formulations.

As used herein, "orally acceptable" refers to a material that is safe and palatable at the relevant concentrations for use in an oral care formulation, such as a mouthwash or dentifrice.

As used herein, "orally acceptable carrier" refers to any vehicle useful in formulating the oral care compositions disclosed herein. The orally acceptable carrier is not harmful to a mammal in amounts disclosed herein when retained in the mouth, without swallowing, for a period sufficient to permit effective contact with a dental surface as required herein. In general, the orally acceptable carrier is not harmful even if unintentionally swallowed. Suitable orally acceptable carriers include, for example, one or more of the following: water, a thickener, a buffer, a humectant, a surfactant, an abrasive, a sweetener, a flavorant, a pigment, a dye, an anti-caries agent, an anti-bacterial, a whitening agent, a desensitizing agent, a

vitamin, a preservative, an enzyme, and mixtures thereof.

The oral care composition is further defined as a product which, during the normal course of usage, is not for the purpose of systemic administration of particular therapeutic agents intentionally swallowed, but is rather retained in the oral cavity for a time sufficient to contact substantially all of the dental surfaces and/or oral tissues for the purposes of oral activity. Examples of such oral care compositions include, but are not limited to toothpaste, dentifrice, mouthwash, mouthrinse, topical oral gel, denture cleanser, dental strips, beads, varnish, dental floss, ribbon toothpaste, chewing gum, lozenges, toothpowder and the like.

As used herein, the term "toothpaste" refers to a paste, a gel, an emulsion, used on a toothbrush for cleaning the teeth. It is understood that a toothpaste for example will typically be diluted with water upon use, while a mouth rinse typically will not be.

As used herein, the term "dentifrice" refers to a paste, gel, or liquid formulations unless otherwise specified. The dentifrice composition may be in any desired form such as deep striped, surface striped, multi-layered, having the gel surrounding the paste, or any combination thereof. Alternatively, the oral care composition can be dual phase dispensed from a separated compartment dispenser.

As used herein, the terms "mouthwash" or "mouthrinse" refer to oral care compositions that are substantially liquid in character, such as a spray, or rinse. In such a preparation, the oral care composition typically has an aqueous phase comprising water or a water and alcohol mixture. Further, in various embodiments, the oral care composition includes a humectant and surfactant as described below. Generally, the weight ratio of water to alcohol is in the range of 1:1 to 20:1, preferably 3:1 to 10:1 and more preferably 4:1 to 6:1. The total amount of water and alcohol mixture in this type of preparation is typically in an amount from 70% by weight to 99.9% by weight of the preparation. In various embodiments, the alcohol is typically ethanol or isopropanol.

As used herein, the term "topical oral gel" refers to a mixture prepared and immediately transferred into a retaining tray, such as those used in holding whitening gels, and the person can wear the tray for an effective period of time. The teeth that are in contact with the mixture will be treated. For use with retaining trays, the mixture can be in the form of a low-viscosity liquid or a gel. In certain embodiments, the whitening agent of the disclosure is formulated in an oral care composition comprising crosslinked copolymer of polyacrylic acid (Carbopol® polymer), glycerin and water.

As used herein, the term "varnish" refers to a stock solution, or a mixture of stock solutions with water, applied to the teeth in a varnish formulation, e.g., wherein the gel can stay on the tooth for an extended period of time for effective treatment. When the oral care composition is in the form of a gel or a varnish, then the oral care composition of the present disclosure is a viscous liquid, preferably a gel, which maintains its consistency during storage enabling the product to be painted on the tooth surface with a soft applicator pen or brush. In certain embodiments, the whitening agent of the disclosure is formulated in an oral care composition comprising hydrophobic copolymers comprising at least one of octylacrylamide /acrylates/ butylaminoethyl, methacrylate copolymer, VA/butyl maleate/isobornyl acrylate copolymer, acrylates/t-butylacrylamide copolymer, polyvinylpyrrolidone/vinyl acetate copolymer, triacontanyl polyvinylpyrrolidone copolymer, acrylates/dimethylaminoethyl methacrylate copolymer (sold under the name of Eudragit®), 2-propenoic acid 2-methyl- 2-methylpropyl ester polymer with 2-propenoic acid and N-(1,1,3,3 -tetramethylbutyl)-2-propenamide (sold under the name of DERMACRYL®), and/or combinations thereof.

As used herein, the term "denture cleanser" refers to a cleaner for dentures when they are out of the mouth. As used herein, the term "dental strips," refers to disposable strips, which contain enamel safe whitening gel, placed directly to the teeth. As used herein, the term "tooth powder" refers to a powder for cleaning the teeth. As used herein, the term "ribbon toothpaste" refers to toothpaste dispensed in the form of a ribbon. As used herein, the term "active", refers to compounds that, when applied to a target tissue, provide a benefit or improvement to the target tissue. The actives can be delivered in the form of any oral care formulations, for example toothpaste, transparent paste, gel, mouthwash, powder, cream, dental strip, spray, gum, or any other known in the art. The term "active" and "agent" are used interchangeably in this disclosure. As used herein, "tooth" or "teeth" refer to natural teeth, dentures, dental plates, fillings, caps, crowns, bridges, dental implants, and the like, and any other hard surfaced dental prosthesis either permanently or temporarily fixed within the oral cavity. As used herein, "whitening" refers to a change in visual appearance of a tooth, wherein the tooth has a brighter shade. As used herein, "applying" refers to any method by which the oral care composition of the disclosure is placed in contact with the tooth surface.

As used herein, one of skill in the art would appreciate that an "ascorbic acid salt" may be referred to by several names in the literature. As used herein, "ascorbic acid derivatives" of the disclosure comprise all the isomers, diastereomers and enantiomers of the cited compounds.

Whitening agents

The disclosure provides for oral care compositions comprising whitening agents comprising ascorbic acid derivatives as further defined in the claims. Generally, ascorbic acid derivatives can be any selected from the group consisting of: L-ascorbic acid, calcium ascorbate, calcium l-ascorbate dihydrate, magnesium ascorbate, potassium

ascorbate, magnesium L-ascorbyl phosphate L-ascorbic acid 2-phosphate sesquimagnesium salt hydrate, (+) sodium L-ascorbate, dehydro-l-(+)-ascorbic acid dimer, sodium ascorbyl phosphate, ascorbic acid-2-glucoside, ascorbyl dipalmitate, ascorbyl methylsilanol pectinate, ascorbyl stearate, disodium ascorbyl sulfate, and ascorbyl 6-palmitate.

The content of ascorbic acid derivative in the oral care composition as defined in the claims is about 1.5% by weight of the total weight of the oral care composition.

In one embodiment, the disclosure provides an oral care composition for whitening teeth, wherein the composition comprises a whitening agent as further defined in the claims.

In one embodiment, the disclosure provides an oral care composition for whitening teeth, wherein the oral care composition comprises a whitening agent which is sodium ascorbyl phosphate in an amount of about 1.5% by weight relative to the total oral care composition as further defined in the claims.

Generally an oral care composition for whitening teeth can be comprising a whitening agent which is ascorbyl 6-palmitate in an amount from 0.5% by weight to 5% by weight relative to the total oral care composition. Generally, an oral care composition for whitening teeth can be comprising a whitening agent which is ascorbyl 6-palmitate in an amount of about 1.5% by weight relative to the total oral care composition. Generally, an oral care composition for whitening teeth can be comprising a whitening agent which is ascorbyl 6-palmitate in an amount of about 2.5% by weight relative to the total oral care composition. Generally, an oral care composition for whitening teeth can be comprising a whitening agent which is ascorbyl 6-palmitate in an amount from 1.0% by weight to 3.0% by weight relative to the total oral care composition.

In one aspect, the oral care compositions of the disclosure are free from peroxides or chemical whitening agents not being an ascorbic acid derivative.

In some embodiments, the oral care composition of the disclosure comprises a further whitening agent, wherein the whitening agent comprises an ascorbic acid salt.

For example, ascorbic acid salts can be selected from the group consisting: calcium ascorbate, calcium l-ascorbate dihydrate, magnesium ascorbate, potassium ascorbate, magnesium L-ascorbyl phosphate L-ascorbic acid 2-phosphate sesquimagnesium salt hydrate, (+) sodium L-ascorbate, dehydro-l-(+)-ascorbic acid dimer, sodium ascorbyl phosphate, ascorbyl dipalmitate, ascorbyl methylsilanol pectinate, ascorbyl stearate, disodium ascorbyl sulfate, and ascorbyl 6-palmitate.

Generally, a whitening agent can comprise an ascorbic acid derivative selected from the group consisting: L-ascorbic acid, calcium ascorbate, calcium l-ascorbate dihydrate, magnesium ascorbate, potassium ascorbate, magnesium L-ascorbyl phosphate L-ascorbic acid 2-phosphate sesquimagnesium salt hydrate, (+) sodium L-ascorbate, dehydro-l-(+)-ascorbic acid dimer, sodium ascorbyl phosphate, ascorbic acid-2-glucoside, ascorbyl dipalmitate, ascorbyl methylsilanol pectinate, ascorbyl stearate, disodium ascorbyl sulfate and ascorbyl 6-palmitate.

The oral care composition of the disclosure comprises a whitening agent as defined in the claims.

In some embodiments, the oral care composition of the disclosure comprises orally acceptable carriers and further comprises one or more ingredients selected from the following: pH modifying agents, erosion prevention agents, foam modulators, thickening agents, flavoring agent, antibacterial agents, desensitizing agents, anticariogenic agents, fluoride ion sources, anticalculus agents, abrasives, stain prevention agents, antitartar agents, polymer complexes, surfactants, humectants and/or combinations thereof.

In some embodiments, the oral care composition of the disclosure comprises a further whitening agent and orally acceptable carriers, and wherein the oral care composition further comprises one or more ingredients selected from the group consisting of: pH modifying agents, erosion prevention agents, foam modulators, thickening agents, flavoring agents, antibacterial agents, desensitizing agents, anticariogenic agents, fluoride ion sources, anticalculus agents, abrasives, stain prevention agents, antitartar agents, polymer complexes, surfactants, humectants and/or combinations thereof.

In a method of utilizing an applicator to deliver the oral care composition of the disclosure, the applicator can be a pen and the pen is stored within an oral care implement. The pen can be removed from the oral care implement prior to application of the oral care composition to the tooth. In some embodiments, the oral care composition may be applied to the tooth after brushing. In some embodiments, the oral care composition may be applied to the tooth after brushing with the oral care implement.

In one embodiment, the present disclosure also provides non-therapeutic methods for whitening a tooth surface using the oral care compositions according to the present disclosure as further defined in the claims.

The method to whiten a surface of a tooth as defined in the claims is comprising: contacting the surface of the tooth with the oral care composition of the disclosure, for the duration of time sufficient to whiten the surface of the tooth as defined in the claims.

The methods described herein may comprise direct application of the oral care composition including rinsing, painting, and brushing. In some embodiments, application of the oral care composition comprises the use of an application device which aids in maintaining contact of the ascorbic acid derivative whitening agent to the tooth surface for sufficient time so as to allow whitening. Suitable application devices include dental trays, mouthpieces, dental floss, fibers, chips, dental strips and tapes. Dental strips comprise polymers, natural and synthetic woven materials, non-woven material, foil,

paper, rubber and/or combinations thereof. In one embodiment, the oral care composition of the disclosure is applied using a "paint on" technique. A small application device, such as a brush or spatula is coated with an oral care composition of the disclosure which is placed on a tooth surface.

In one embodiment, the oral care composition of the disclosure is in the form selected from: dentifrice (e.g., toothpaste), mouthwash, mouth rinse, topical oral gel, denture cleanser, dental strips, beads, varnish, dental floss, tablets, ribbon strips, ribbon toothpaste, chewing gum, lozenges and toothpowder.

The oral care composition of the disclosure comprises an ascorbic acid derivative as further defined in the claims, and can further comprises a fluoride ion source.

The oral care composition of the disclosure comprises an ascorbic acid derivative as further defined in the claims, and can further comprise a zinc ion source.

The oral care composition of the disclosure comprises one or more ascorbic acid derivatives as further defined in the claims, and can further comprise a stannous ion source.

**Oral care composition ingredients**

In one embodiment, the oral care compositions of the disclosure may comprise at least one abrasive agent in addition to an ascorbic acid derivative as defined in the claims. The role of an abrasive is to act as a polishing agent within the oral care composition. In some embodiments, the abrasive agents include without limitation silica (in the form of silica gel, hydrated silica, perlite, high cleaning silica, core shell silica or precipitated silica), alumina, insoluble phosphates, calcium carbonate, resinous abrasives such as urea-formaldehyde condensation products, dicalcium phosphate, calcium pyrophosphate, calcium carbonate, sodium bicarbonate, alumina, dicalcium orthophosphate dihydrate, n-calcium pyrophosphate, tricalcium phosphate, calcium polymetaphosphate, insoluble sodium polymetaphosphate, precipitate calcium carbonate. The average particle size of the abrasive is generally about 0.1 to about 30 $\mu$m for example about 1 to about 20 $\mu$m or about 5 to about 15 $\mu$m. In some embodiments, one or more abrasives are present in an amount of about 0.1 % by weight to about 60% by weight of the total weight of the oral care composition. In some embodiments, the abrasive is calcium pyrophosphate. In some embodiments, the calcium pyrophosphate is present in an amount from about 5% to about 50% by weight of the total weight of the oral care composition. In some embodiments the abrasive is perlite, sold under the name of Perlite ImerCare® 25P. In some embodiments the abrasive is core shell silica. In some embodiments the abrasive is precipitated calcium carbonate. In some embodiments the oral care composition of the disclosure comprises an abrasive comprising cellulose, such as non-colloidal microcrystalline cellulose, colloidal microcrystalline cellulose and/or combinations thereof.

In one embodiment, the oral care compositions of the disclosure may comprise at least one stain prevention agent in addition to an ascorbic acid derivative as defined in the claims. In some embodiments the stain prevention agents include tetrasodium pyrophosphate (TSPP), disodium pyrophosphate (SAPP), sodium tripyrophosphate (STPP), sodium hexametaphosphate (SHMP), or zinc citrate.

In some embodiments, one or more stain prevention agents are present in an amount of about 0.1 % to about 60% by weight of the total weight of the oral care composition.

The composition as defined in the claims comprises a zwitterionic surfactant. In one embodiment, the oral care compositions of the disclosure may comprise at least one further surfactant agent in addition to an ascorbic acid derivative as defined in the claims. The role of the surfactant is to enhance the stability of the oral care composition and create foam during stirring which ensures cleaning the oral cavity. In some embodiments the surfactant agents comprise anionic, nonionic or amphoteric surfactants.

Suitable anionic surfactants include without limitation water-soluble salts of $C_{8-20}$ alkyl sulfates, sulfonated monoglycerides of $C_{8-20}$ fatty acids, sarcosinates, taurates and the like. Illustrative examples of these and other classes include sodium cocoyl monoglyceride sulfonate, sodium stearate, sodium lauryl sarcosinate (sold under the name of Maprosyl ®30-B), lauryl glucoside, sodium lauryl isoethionate (sold under the name of Tauranol ®WHSP), sodium laureth carboxylate, sodium lauryl sulfate (same as sodium dodecyl sulfate or SLS or SDS) and sodium dodecyl benzenesulfonate.

Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation derivatives of $C_{8-20}$ aliphatic secondary and tertiary amines having an anionic group such as carboxylate, sulfate, sulfonate, phosphate, phosphonate or cocoamidopropyl betaine, alkyl betaines and alkyl amido betaines.

In some embodiments the surfactant is a mixture of sodium cocoamphoacetate and glycerin and lauryl glucoside and sodium cocoyl glutamate, sold under the name of Plantapon®, and sodium lauryl glucose carboxylate. In some embodiments the surfactant is sodium carboxymethyl cellulose, sodium stearate, lauryl glucoside, polyanionic cellulose, sodium lauryl isoethionate, sodium lauryl sarcosinate, sodium cocoyl glutamate, cocamidopropyl betaine, and/or combinations thereof. In some embodiments, one or more surfactants may be present in a total amount of from about

0.01% by weight to about 20.00% by weight of the total weight of the oral care composition. In some embodiments, one or more surfactants may be present in a total amount of from about 1.8% by weight to about 2% by weight of the total weight of the oral care composition. In some embodiments, one or more surfactants may be present in a total amount of from about 1.9% to about 2% by weight of the total weight of the oral care composition. In some embodiments, one or more surfactants may be present in a total amount of about 2% by weight of the total weight of the oral care composition.

In one embodiment, the oral care compositions of the disclosure may comprise at least one thickening agent in addition to an ascorbic acid derivative as defined in the claims. In some embodiments the thickening agents include without limitation: carbomers, also known as carboxyvinyl polymers, carrageenans, also known as Irish moss and more particularly carrageenan (iota-carrageenan), high molecular weight polyethylene glycols (such as CARBOWAX®, available from The Dow Chemical Company), cellulosic polymers such as hydroxyethylcellulose, carboxymethyl cellulose (CMC) and salts thereof, CMC sodium, polyanionic cellulose, natural gums such as karaya, xanthan gum, thickening silica, gum arabic and tragacanth, microcrystalline cellulose, colloidal magnesium aluminum silicate, and colloidal silica, fumed silica and/or combinations of the same.

In some embodiments the thickening agent is thickening silica or xanthan gum and/or a combination thereof. In some embodiments, the thickening agent is present in a total amount of about 0.1% by weight to about 90% by weight of the total weight of the oral care composition. In some embodiments, the one or more optional thickening agents are present in a total amount of about 1 % by weight to about 50% by weight of the total weight of the oral care composition. In some embodiments, the one or more optional thickening agents are present in a total amount of about 5% by weight to about 35% by weight of the total weight of the oral care composition.

In one embodiment, the oral care compositions of the disclosure may comprise at least one humectant agent in addition to an ascorbic acid derivative as defined in the claims. In some embodiments the humectant agents include polyhydric alcohols such as vegetable refined glycerin, non-crystal sorbitol, xylitol or low molecular weight polyethylene glycols (PEGs) or polyoxyethylenes. In some embodiments, the humectant agent is non crystal sorbitol, vegetable glycerin or polyoxyethylene glycol and/or a combination thereof. High molecular weight PEGs are suitable, including those having an average molecular weight of about 200,000 to about 7,000,000, for example about 500,000 to about 5,000,000 or about 1,000,000 to about 2,500,000. One or more PEGs are optionally present in a total amount of about 0.1% to about 10%, for example about 0.2% by weight to about 5% by weight or about 0.25% by weight to about 2% by weight of the total weight of the oral care composition.

The oral care composition of the disclosure may comprise at least one adhesion agent. The role of the adhesion agent is to ensure the retention of the oral care composition on the oral cavity surface. In some embodiments, the adhesion agents include adhesives, film forming materials, viscosity enhancers, hydrophilic organic polymers, hydrophobic organic polymers, silicone gums, silicone adhesives, silicas, and/or combinations thereof. Adhesion agents are preferably present at a level of from about 0.01 % by weight to about 75% by weight, optionally from about 1 % by weight to about 40% by weight of the total weight of the oral care composition.

In one embodiment, the oral care compositions of the disclosure may comprise at least one hydrophobic polymer carrier in addition to an ascorbic acid derivative as defined in the claims, and wherein the oral care composition is free of water. The term hydrophobic or water-insoluble as applied to polymers and as employed herein refers to polymers which are substantially non-aqueous having a water solubility of less than one gram per 100 grams of water at 25° C. In certain embodiments, compositions of the disclosure comprise at least one hydrophobic polymer carrier, wherein the hydrophobic polymer carrier is a silicone pressure sensitive adhesive, and at least one whitening agent, wherein the whitening agent is an ascorbic acid derivative. For example, the ascorbic acid derivative may be present in the oral care composition in an amount ranging from about 0.5 % by weight to about 5.0% by weight relative to the total weight of the oral care composition.

In various embodiments, a hydrophobic polymer is compatible with the at least one whitening agent described herein as defined in the claims. In certain embodiments, a hydrophobic polymer is selected for the carrier to produce a tooth whitening oral care composition having a viscosity ranging from about 1,000 cPs to about 900,000 cPs, such as from about 10,000 cPs to about 900,000 cPs or from about 10,000 cPs to about 100,000 cPs. One class of hydrophobic polymers that may be used according to certain exemplary embodiments, comprise siloxane polymers, which are also generally known in the art as "silicone" polymers, such as silicone pressure sensitive adhesives (PSA). In certain embodiments disclosed herein, the hydrophobic polymers in the carrier are those in which a whitening agent can be dispersed and are well known in the art. For example, silicone polymers may be commercially available. In various embodiments, a silicone-based hydrophobic polymer is a polyorganosiloxane, such as polydimethylsiloxane. In one embodiment, the oral care compositions disclosed herein may further comprise crospovidone (poly[N-vinyl-2-pyrrolidone]) as an adhesion enhancing agent. Crospovidone may be present in the oral care composition in an amount ranging from about 10% by weight to about 30%, by weight relative to the total weight of the tooth whitening oral care composition, such as ranging from about 15% by weight to about 25% by weight, or ranging from about 18% by weight to about 25% by weight of the total weight of the oral care composition. In another embodiment the oral care compositions disclosed herein may further comprise N-vinyl heterocyclic polymers. The N-vinyl heterocyclic polymers are derived from N-heterocyclic vinyl monomers, or specific monomers such as N-vinyl imides or N-vinyl lactams. One preferred polymer is polyN-vinyl-poly-2-pyrrolidone (PVP).

In another embodiment, the oral care composition of the disclosure may comprise at least one antioxidant agent. In some embodiments, the antioxidant agent(s) maybe be selected from the group consisting of: butylated hydroxyanisole (BHA), butylated hydroxytoluene (BHT), vitamin A, carotenoids, quercetin, rutin, catechin, trolox, vitamin E, flavonoids, polyphenols, ascorbic acid, 2,4-dihydroxybenzoic acid, m-methoxybenzoic acid, m-hydroxybenzoic acid, p-hydroxybenzoic acid, 3,4-dihydroxybenzoic acid, stannous compounds, potassium or sodium meta-bisulfite, butylated hydroxytoluene (BHT), ammonium sulfate, herbal antioxidants, chlorophyll, melatonin, and/or combinations thereof.

In another embodiment, the oral care composition of the disclosure may comprise at least one erosion prevention agent selected from the group consisting of: sodium fluoride, arginine, arginine phosphate, arginine hydrochloride, arginine bicarbonate, sodium monofluorophosphate, potassium citrate, titanium phosphate, zinc oxide, zinc citrate, zinc citrate trihydrate, zinc lysine chloride complexes, zinc arginine chloride complexes are erosion prevention agents. The erosion prevention agent or combinations thereof may be present in the oral care composition in an amount ranging from about 0.2 % by weight to about 10.0% by weight relative to the total weight of the oral care composition.

In another embodiment, the oral care composition of the disclosure may comprise at least one preservative agent selected from the group consisting of: benzyl alcohol, chlorhexidine, benzoic acid, benzoic acid salts, quaternary ammonium compounds (such as benzalkonium chloride) are preservative agents.

In another embodiment, the oral care composition of the disclosure may comprise at least one antimicrobial agent selected from the group consisting of: triclosan (5-chloro-2-(2,4-dichlorophenoxy)phenol); 8-hydroxyquinoline and salts thereof; zinc and stannous ion sources such as zinc citrate, zinc sulphate, zinc glycinate, sodium zinc citrate and stannous pyrophosphate; copper (II) compounds such as copper (II) chloride, fluoride, sulfate and hydroxide; phthalic acid and salts thereof such as magnesium monopotassium phthalate; sanguinarine; quaternary ammonium compounds, such as alkylpyridinium chlorides, cetylpyridinium chloride (CPC), combinations of CPC with zinc and/or enzymes, tetradecylpyridinium chloride, and N-tetradecyl-4-ethylpyridinium chloride; bisguanides, such as chlorhexidine digluconate, hexetidine, octenidine, and alexidine; halogenated bisphenolic compounds, such as 2,2' methylenebis-( 4-chloro-6-bromophenol); benzalkonium chloride; salicylanilide, domiphen bromide; iodine; sulfonamides; bisbiguanides; phenolics; piperidine derivatives such as delmopinol and octapinol; magnolia extract; grapeseed extract; thymol; eugenol; menthol; geraniol; carvacrol; citral; eucalyptol; catechol; 4-allylcatechol; hexyl resorcinol; methyl salicylate; antibiotics such as augmentin, amoxicillin, tetracycline, doxycycline, minocycline, metronidazole, neomycin, kanamycin and clindamycin; and/or combinations thereof.

In another embodiment, the oral care composition of the disclosure may comprise at least one antitartar (anticalculus) agent selected from the group consisting of: phosphates and polyphosphates (for example pyrophosphates), polyaminopropanesulfonic acid (AMPS), polyolefin sulfonates, polyolefin phosphates, diphosphonates such as azacycloalkane- 2,2-diphosphonates ( e.g., azacycloheptane-2,2-diphosphonic acid), N-methyl azacyclopentane-2,3-diphosphonic acid, ethane-1-hydroxy-1, 1-diphosphonic acid (EHDP) and ethane-1-amino-1,1-diphosphonate, phosphonoalkane carboxylic acids and salts of any of these agents, for example their alkali metal and ammonium salts. Useful inorganic phosphate and polyphosphate salts include monobasic, dibasic and tribasic sodium phosphates, sodium tripolyphosphate, tetrapolyphosphate, mono-, di-, tri- and tetrasodium pyrophosphates, sodium trimetaphosphate, sodium hexametaphosphate and/or combinations thereof, wherein sodium may optionally be replaced by potassium or ammonium. Anticalculus agents include polycarboxylate polymers and polyvinyl methyl ether/maleic anhydride (PVME/MA) copolymers, such as those available under the Gantrez™ brand from ISP, Wayne, N.J. In another embodiment, the oral care compositions of the disclosure comprise one or more anticalculus agents present in an effective total amount, e.g., 0.01 % by weight to 50% by weight, for example 0.05% by weight to 25% by weight, or 0.1% by weight to 15% by weight of the total weight of the oral care composition.

In another embodiment, the oral care composition of the disclosure may comprise at least one stannous ion source. Stannous ions may act as a periodontal active, antitartar agent, anticariogenic agent or tooth desensitizer. In some embodiments, the stannous ion source can be selected from the group consisting of: stannous fluoride, other stannous halides such as stannous chloride dihydrate, organic stannous carboxylate salts such as stannous formate, acetate, gluconate, lactate, tartrate, oxalate, malonate and citrate, stannous ethylene glyoxide, and/or combinations thereof.

In another embodiment, the oral care composition of the disclosure may comprise at least one fluoride ion source. Fluoride ions may act as an anticariogenic agent. In some embodiments, the fluoride ion source can be selected from the group consisting of: potassium, sodium and ammonium fluorides and monofluorophosphates, sodium monofluorophosphates, stannous fluoride, indium fluoride, and/or combinations thereof.

In another embodiment, the oral care composition of the disclosure may comprise at least one zinc ion source. Zinc ions may act as antimalodor agents. In some embodiments, the zinc ion source can be selected from the group consisting of: zinc oxide, zinc citrate, zinc chloride, zinc acetate, zinc lactate, zinc salicylate, zinc sulfate, zinc phosphate, zinc tartrate and zinc nitrate and/or combinations thereof.

In another embodiment, the oral care composition of the disclosure may comprise at least one colorant source. In some embodiments, the colorant source can be selected from the group consisting of: pigments, dyes, lakes and agents imparting a particular luster or reflectivity such as pearling agents. In various embodiments, colorants are operable to

provide a white or light-colored coating on a dental surface, to act as an indicator of locations on a dental surface that have been effectively contacted by the oral care composition, and/or to modify appearance, in particular color and/or opacity, of the oral care composition to enhance attractiveness to the consumer.

In another embodiment, the oral care composition of the disclosure may comprise at least one flavoring agent. For example, the oral care compositions of the disclosure may comprise 0.1 % by weight to 2.0% by weight of flavoring agent, of the total weight of the oral care composition. In some embodiments, the flavoring agent can be any material and/or combinations of materials operable to enhance the taste of the oral care composition, e.g., any orally acceptable natural or synthetic flavoring agent (e.g., flavoring oils, flavoring aldehydes, esters, alcohols, similar materials, and/or combinations thereof). Flavoring agents include vanillin, sage, marjoram, parsley oil, spearmint oil, cinnamon oil, oil of wintergreen (methyl salicylate) peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, bean- and nut-derived flavors such as coffee, cocoa, cola, peanut, almond, adsorbed and encapsulated flavoring agents and/or combinations thereof. Also encompassed within flavoring agents herein are ingredients that provide other sensory effects in the mouth, including cooling or warming effects.

In another embodiment, the oral care compositions of the disclosure may comprise at least one sweetener. The oral care composition of the disclosure may comprise from 0.1 % by weight to 2.0% by weight of sweetener of the total weight of the oral care composition. In some embodiments, the sweetener includes dextrose, sucrose, polydextrose, maltose, dextrin, dried invert sugar, mannose, xylose, ribose, fructose, levulose galactose, corn syrup, partially hydrolyzed starch, hydrogenated starch hydrolysate, sorbitol, mannitol, xylitol, maltitol, isomalt, aspartame, neotame, saccharin and salts thereof, sucralose, dipeptide-based intense sweeteners, cyclamates, dihydrochalcones, stevia, glycosilated stevia glucosides and/or combinations thereof.

In another embodiment, the oral care compositions of the disclosure may comprise at least one mouth-feel agent. In some embodiments, the mouth-feel agents include materials imparting a desirable texture or other feeling during use of the oral care composition of the disclosure.

In another embodiment, the oral care compositions of the disclosure may comprise at least one pH modifying agent. The role of the pH modifying agent is to keep the oral care compositions at an orally acceptable pH range. In some embodiments, the pH modifying agents include acidifying agents to lower pH, basifying agents to raise pH, and buffering agents to control pH within a desired range. Orally acceptable pH modifying agents include carboxylic, phosphoric and sulfonic acids, acid salts, monosodium citrate, disodium citrate, monosodium malate, alkali metal hydroxides such as sodium hydroxide, carbonates such as sodium carbonate, bicarbonates, sesquicarbonates, borates, silicates, phosphates, monosodium phosphate, trisodium phosphate, pyrophosphate salts, imidazole, and/or combinations thereof. The oral care composition of the disclosure may comprise a viscosity modifier. The role of the viscosity modifier is to ensure the oral care composition is homogenous. In some embodiments, the viscosity modifiers include mineral oil, petrolatum, clays and organomodified clays, silica, and/or combinations thereof.

In another embodiment, the oral care compositions of the disclosure may comprise at least one foam modulator. The role of the foam modulator is to generate foam upon mixing which contributes to the dispersion of the oral care composition in the oral cavity. In some embodiments, the foam modulators include sodium lauroyl sarcosinate, sodium lauryl sulfate powder, polyethylene glycols (PEGs), also known as polyoxyethylenes. High molecular weight PEGs are suitable, including those having an average molecular weight of about 200,000 to about 7,000,000, for example about 500,000 to about 5,000,000 or about 1,000,000 to about 2,500,000. One or more PEGs are optionally present in a total amount of about 0.1% by weight to about 10% by weight, for example about 0.2% by weight to about 5% by weight or about 0.25% by weight to about 2% by weight of the total weight of the oral care composition.

In another embodiment, the oral care compositions of the disclosure may comprise at least one saliva stimulating agent. The role of the saliva stimulating agent is to keep the mouth from drying. In some embodiments, the saliva stimulating source includes food acids such as citric, lactic, rilalic, succinic, ascorbic, adipic, fumaric and tartaric acids, and/or combinations thereof.

In another embodiment, the oral care compositions of the disclosure may comprise at least one freshening agent. In some embodiments, the breath freshening agent includes zinc salts such as zinc gluconate, zinc citrate and zinc chlorite, a-ionone, and/or combinations thereof.

In another embodiment, the oral care compositions of the disclosure may comprise at least desensitizing agent. In some embodiments, the desensitizing agents include potassium citrate, potassium chloride, potassium tartrate, potassium bicarbonate, potassium oxalate, potassium nitrate, strontium salts, and/or combinations thereof.

In another embodiment, the oral care compositions of the disclosure may comprise at least one nutrient agent. In some embodiments, the nutrient includes vitamins, minerals, amino acids, and/or combinations thereof. Vitamins include vitamin D, thiamine, riboflavin, calcium pantothenate, niacin, folic acid, nicotinamide, pyridoxine, cyanocobalamin, para-aminobenzoic acid, bioflavonoids, and/or combinations thereof. Nutritional supplements include amino acids, arginine, L-tryptophane, L-lysine, methionine. threonine, levocarnitine and L-carnitine , lipotropics, choline, inositol, betaine, and linoleic acid, fish oil, including components thereof such as omega-3 (N-3) polyunsaturated fatty acids, eicosapentaenoic

acid and docosahexaenoic acid, coenzyme Q10, and/or combinations thereof. The oral care composition of the disclosure may comprise neutral amino acids, wherein the neutral amino acid is selected from the group consisting of alanine, aminobutyrate, asparagine, cysteine, cystine, glutamine, glycine, hydroxyproline, isoleucine, leucine, methionine, phenylalanine, proline, serine, taurine, threonine, tryptophan, tyrosine, valine, and/or combinations thereof; wherein preferably selected from glycine, asparagine, glutamine, and/or combinations thereof; and wherein the neutral amino acid is present in the amount of from 0.1% by weight to 5% by weight of the total weight of the oral care composition.

In another embodiment, the oral care compositions of the disclosure may comprise at least one anti-inflammatory agent. In some embodiments, the anti-inflammatory agents include steroidal agents such as fluocinolone and hydrocortisone, and nonsteroidal agents (NSAIDs) such as ketorolac, flurbiprofen, ibuprofen, naproxen, indomethacin, diclofenac, etodolac, indomethacin, sulindac, tolmetin, ketoprofen, fenoprofen, piroxicam, nabumetone, aspmn, diflunisal, meclofenamate, mefenamic acid, oxyphenbutazone and phenylbutazone, and/or combinations thereof.

In some embodiments, the oral care compositions of the disclosure may comprise at least one ingredient selected from the following: a humectant, an abrasive, a surfactant, a thickening agent, a foam modulator, a stain prevention agent, a preservative, an erosion prevention agent, an antiplaque agent, a sweetener, water, and combinations thereof.

In some embodiments, the oral care compositions of the disclosure may comprise at least one humectant from 0.10% by weight to 70% by weight, at least one abrasive from about 0.10% by weight to about 60% by weight, at least one anionic surfactant from 0.01 % by weight to 20% by weight, at least one a nonionic surfactant from 0.01% by weight to 20% by weight, at least one amphoteric surfactant from 0.01% by weight to 20% by weight, at least one thickening agent from 0.10% by weight to 90% by weight, at least one foam modulator from 0.10% by weight to 10% by weight, at least one stain prevention agent from 0.10% by weight to 60% by weight, at least one preservative from 0.20% by weight to 1% by weight, at least one erosion prevention agent 0.10% by weight to 10% by weight, at least one antiplaque agent from 0.01% to 5% by weight, at least one sweetener from 0.10% by weight to 2% by weight, and/or combinations thereof, wherein the weight is based on the total weight of the composition.

In some embodiments the oral care composition of the disclosure may comprise at least one humectant from 0.10% by weight to 70% by weight, or from 0.10 % by weight to 1% by weight, or from 1% by weight to 5.5% by weight, or from 5.5% by weight to 20% by weight, or from 20% by weight to 40% by weight, or from 40% by weight to 46% by weight, or from 45% by weight to 53% by weight, or from 50% by weight to 60% by weight, or from 61.00% by weight to 68.00% by weight, relative to the total oral care composition.

In some embodiments the oral care composition of the disclosure may comprise at least one abrasive from 0.10% by weight to 60% by weight, or from about 0.10 % by weight to 10% by weight, or from 5% by weight to 7.5% by weight, or from about 10.00 % by weight to 15.00% by weight, or from 15.00 % by weight to 21.00% by weight, or from 21.00 % by weight to 35.00% by weight, or from 7.00 % by weight to 17.00 % by weight, or from 35.00% by weight to 51.00 % by weight, or from 51.00% by weight to 60.00% by weight, relative to the total oral care composition.

In some embodiments the oral care composition of the disclosure may comprise at least one anionic surfactant from 0.01% by weight to 20% by weight, or from 0.01 % by weight to 0.7% by weight, or from 0.45 % by weight to 0.7 % by weight, or from 0.7% by weight to 1 % by weight, or from 1.00% by weight to 10% by weight, or from 10% by weight to 15.5% by weight, or from 4.2% by weight to 5% by weight, or from 15.5% by weight to 20% by weight, relative to the total oral care composition.

In some embodiments the oral care composition of the disclosure may comprise at least one nonionic surfactant from 0.01% by weight to 20% by weight, or from 0.01 % by weight to 0.05% by weight, or from 0.04 % by weight to 1% by weight, or from 1% by weight to 3% by weight, or from 3% by weight to 5% by weight, or from 5% by weight to 7% by weight, or from 7% by weight to 14% by weight, or from 14% by weight to 20% by weight, relative to the total oral care composition.

In some embodiments the oral care compositions of the disclosure may comprise at least one amphoteric surfactant from 0.01% by weight to 20% by weight, or from 0.01 % by weight to 1.00% by weight, or from 1.00 % by weight to 1.6% by weight, or from 1.5% by weight to 2% by weight, or from 2% by weight to 2.6% by weight, or from 2.5% by weight to 3% by weight, or from 3% by weight to 5% by weight, or from 5% by weight to 7% by weight, or from 7% by weight to 14% by weight, or from 14% by weight to 20% by weight, relative to the total oral care composition.

In some embodiments the oral care compositions of the disclosure may comprise at least one thickening agent from 0.1% by weight to 90% by weight, or from 0.1% by weight to 5.5% by weight, or from 5.5% by weight to 7.5% by weight, or from 7.5% by weight to 15.5% by weight, or from 1.5% by weight to 5.5% by weight, or from 0.2% by weight to 0.5% by weight, or from 0.4% by weight to 1.5% by weight, or from 1.5% by weight to 2.5% by weight, or from 2.5% by weight to 3.5% by weight, or from 3.5% by weight to 5% by weight, or from 5% by weight to 5.5% by weight, or from 5.5% by weight to 7% by weight, or from 7% by weight to 8% by weight, or from 7.2 % by weight to 12.8% by weight, or from 15.5% by weight to 35.5% by weight, or from 35.5% by weight to 55.5% by weight, or from 55.5% by weight to 75.5% by weight, or from 75.5% by weight to 85.5% by weight, or from 85.5% by weight to 90% by weight, relative to the total oral care composition.

In some embodiments the oral care compositions of the disclosure may comprise at least one foam modulator from 0.10% by weight to 10% by weight, or from 1% by weight to 2% by weight, or from 2% by weight to 3.5% by weight, or

from 3.5% by weight to 5% by weight, or from 5% by weight to 6% by weight, relative to the total oral care composition. The oral care composition of the disclosure may comprise at least one stain prevention agent from 0.1% by weight to 60% by weight, or from 0.1 % by weight to 0.45% by weight, or from 0.45 % by weight to 5.45% by weight, or from 5.45 % by weight to 10.65 % by weight, or from 10.65% by weight to 20.85 % by weight, or from 20.85% by weight to 40% by weight, or from 40% by weight to 50% by weight, or from 50% by weight to 60% by weight, relative to the total oral care composition.

In some embodiments the oral care compositions of the disclosure may comprise at least one preservative from 0.20% by weight to 1% by weight, or from 0.2% by weight to 0.45% by weight, or from 0.45 % by weight to 0.55 % by weight, or from 0.55% by weight to 0.85 % by weight, or from 0.85% by weight to 1% by weight, relative to the total oral care composition.

In some embodiments the oral care compositions of the disclosure may comprise at least one erosion prevention agent from 0.10% by weight to 10% by weight, or from 0.55% by weight to 0.85 % by weight, or from 0.10 % by weight to 0.2% by weight, or from 0.2% by weight to 0.5% by weight, or from 0.2% by weight to 0.35% by weight, or from 0.35 % by weight to 0.65 % by weight, or from 0.65% by weight to 0.85 % by weight, or from 0.85% by weight to 1% by weight, relative to the total oral care composition.

In some embodiments the oral care composition of the disclosure may comprise at least one antiplaque agent from 0.01% by weight to 50% by weight, or from 0.01 % by weight to 0.5% by weight, or from 0.5% by weight to 1% by weight, or from 1% by weight to 5% by weight, or from 5% by weight to 10% by weight, or from 10% by weight to 20% by weight, or from 20% by weight to 30.2% by weight, or from 30.2% by weight to 40% by weight, or from 40% by weight to 50% by weight, relative to the total oral care composition.

The oral care composition of the disclosure may comprise at least one sweetener from 0.10% by weight to 2% by weight, or from 0.1% by weight to 0.4% by weight, or from 0.4 % by weight to 1% by weight, or from 1% by weight to 1.2% by weight, or from 1.2% by weight to 1.7% by weight, or from 1.7% by weight to 2% by weight, relative to the total oral care composition.

It is understood that while general attributes of each of the above categories of materials may differ, there may be some common attributes and any given material may serve multiple purposes within two or more of such categories of materials.

In certain embodiments, the oral care composition is anhydrous. Anhydrous, refers to less than 5% by weight of water, optionally less than 4%, less than 3%, less than 2% by weight, less than 1% by weight, less than 0.5% by weight, less than 0.1% by weight down to 0% by weight water.

In certain embodiments, the oral care compositions of the disclosure comprise water in an amount of 60% by weight to 90% by weight of the total weight of the oral care composition. In certain embodiments, the oral care composition comprises water in an amount of 65% by weight to 85% by weight of the total weight of the oral care composition. In certain embodiments, the oral care composition comprises water in an amount of 70% by weight to 80% by weight of the total weight of the oral care composition. In certain embodiments, the oral care composition comprises water in an amount of 10% by weight to 22% by weight of the total weight of the oral care composition. In certain embodiments, the oral care composition comprises water in an amount of 5% by weight to 30% by weight of the total weight of the oral care composition.

The oral care compositions of the disclosure may comprise various agents which are active to protect and enhance the strength and integrity of the enamel and tooth structure and/or to reduce bacteria and associated tooth decay and/or gum disease. Effective concentration of the active ingredients used herein will depend on the particular agent and the delivery system used.

In one aspect, the present disclosure therefore provides an oral care composition as defined in the claims.

The amount of the ascorbic acid derivative can be sufficient to increase ΔW of the enamel by at least 0.3 times relative to a non-peroxide reference standard.

The amount of the ascorbic acid derivative can be sufficient to increase ΔW of the enamel by 0.3 - 4 times greater relative to a non-peroxide reference standard (e.g., about 0.5 times) (e.g., about 2.5 times greater).

The composition can be comprising a stannous ion source.

The stannous ion source can be selected from the group consisting of: stannous fluoride, stannous chloride, stannous pyrophosphate, stannous formate, stannous acetate, stannous gluconate, stannous wt. lactate, stannous tartrate, stannous oxalate, stannous malonate, stannous citrate, stannous ethylene glyoxide, and combinations thereof.

The stannous ion source can comprise stannous fluoride.

The stannous ion source can comprise stannous fluoride in an amount of 0.1 wt. % to 2 wt. % (0.1 wt.% - 0.6 wt.%) (e.g., about 0.454 wt.%) of the total composition weight.

The stannous ion source can comprise stannous fluoride in an amount from 50 to 25,000 ppm (e.g., 750 -7000ppm, e.g., 1000-5000ppm, e.g., about 4500 ppm, e.g., about 4540ppm).

The composition can comprise stannous fluoride and stannous pyrophosphate.

The composition can comprise stannous fluoride and stannous chloride.

The one or more stannous ion source(s) can be present in an amount from 0.1% - 5% by wt. of the total

composition.

The composition can comprise a zinc ion source and wherein the zinc ion source comprises one or more zinc salt(s) selected from the group consisting of: zinc citrate, zinc oxide, zinc phosphate, zinc lactate, zinc sulfate, zinc silicate, zinc gluconate and combinations thereof.

The composition can comprise a zinc ion source and wherein the zinc ion source comprises zinc oxide.

The composition can comprise a zinc ion source and wherein the zinc ion source comprises zinc citrate.

The composition can comprise a zinc ion source and wherein the zinc ion source comprises zinc oxide and zinc citrate.

The ratio of the amount of zinc oxide (e.g., wt.%) to zinc citrate (e.g., wt.%) can be from 1.5:1 to 4.5:1 (e.g., 2:1, 2.5:1, 3:1, 3.5:1, or 4:1).

The zinc citrate can be present in an amount of from 0.25 to 0.75 wt.% (e.g., 0.5 wt. %) and zinc oxide may be present in an amount of from 0.75 to 1.25 wt.% (e.g., 1.0 wt. %) based on the weight of the oral care composition.

Zinc citrate can be about 0.5 wt.%.

Zinc oxide can be about 1.0 wt.%.

Zinc citrate can be about 0.5 wt.% and the zinc oxide is about 1.0 wt.%.

The composition can comprise a zinc ion source and wherein the zinc ion source can comprise zinc phosphate (e.g., wherein the zinc phosphate is a preformed salt of zinc phosphate) (e.g., zinc phosphate hydrate) (e.g., from 0.5 - 4wt% of zinc phosphate) (e.g., about 1.0 wt.% of zinc phosphate).

Zinc phosphate can be added as a pre-formed salt.

The composition can comprise a source of zinc ions, and wherein the zinc ion source can comprise zinc lactate.

The zinc ion source can be in an amount from 0.1% - 5% by wt. of the total composition (e.g., zinc phosphate from 0.1% - 5% by wt. of the total composition). The composition can be comprising an effective amount of a fluoride ion source. The amount of the fluoride ion source can be in an amount from 0.01% to 5% by weight, relative to the weight of the oral care composition, for example, from 0.05 to 4% by weight, or from 0.1% to 3% by weight, or from 0.2 to 2% by weight, or from 0.3 to 1% by weight, or from 0.3 to 0.5% by weight, or about 0.32% by weight (e.g., 0.32% by weight).

The fluoride source can be selected from the group consisting of: sodium fluoride, potassium fluoride, calcium fluoride, zinc fluoride, zinc ammonium fluoride, lithium fluoride, ammonium fluoride, stannous fluoride, stannous fluorozirconate, sodium monofluorophosphate, potassium monofluorophosphate, laurylamine hydrofluoride, diethylaminoethyloctoylamide hydrofluoride, didecyldimethylammonium fluoride, cetylpyridinium fluoride, dilaurylmorpholinium fluoride, sarcosine stannous fluoride, glycine potassium fluoride, glycine hydrofluoride, amine fluorides and combinations thereof.

The fluoride ion source can comprise sodium fluoride (e.g., from 0.2% - 2% by wt. of sodium fluoride)

The fluoride ion source can comprise stannous fluoride (e.g., stannous fluoride from 0.1% - 2% by wt. of the total composition).

The fluoride ion source can comprise sodium monofluorophosphate.

The composition can comprise water in the amount of 10% by weight or more, relative to the weight of the oral care composition, for example, 10-90%, or 10-80%, or 10-70%, or 10-60%, or 10-50%, or 10-40%, or 10-30%, or 15-30%, 15% -40% 20% -40%, 20-35%, or 20-50%, or 30-35%, or about 25% or about 30%, by weight of the composition.

The composition can be further comprising an organic buffer system, wherein the buffer system comprises a carboxylic acid and one or more conjugate base salts thereof, for example, alkali metal salts thereof (e.g., citric acid and sodium citrate). The composition can comprise the organic acid buffer system in an amount of 0.1 to 5.0% by weight of the composition, measured as the combined amount of organic acid and any conjugate base salts (e.g., citric acid and sodium citrate); for example, from 0.5 to 4.0%, or from 1.0 to 3.0%, or from 1.5 to 3.0%, or from 1.0 to 2.4%, or from 1.0% to 2.0%, or from 1.0% to 1.5%, or about 1.2%, by weight of the composition.

The oral care composition can further comprise an abrasive, for example, silica abrasives, calcium abrasives, and other abrasives as disclosed herein.

The composition can be further comprising one or more humectants, as described herein, e.g., selected from sorbitol, glycerol, xylitol and propylene glycol, or combinations thereof, e.g., a combination of sorbitol and glycerin.

The zwitterionic surfactant can comprise cocamidopropyl betaine, (e.g., in an amount of 0.1-5% by weight) (e.g., about 0.6% by wt.).

The composition can be further comprising an effective amount of one or more alkali phosphate salts for example orthophosphates, pyrophosphates, tripolyphosphates, tetraphosphates or higher polyphosphates.

The alkali phosphate salts can comprise tetrasodium pyrophosphate or tetrapotassium pyrophosphate, for example, in an amount of 0.5 to 5% by weight of the composition, e.g., 1-4%, or about 2-4%, or about 1-2% or about 1.5% or about 2% or about 4%, by weight.

The alkali phosphate salts can comprise sodium tripolyphosphate or potassium tripolyphosphate, for example, in an amount of 0.5 to 6% by weight of the composition, e.g., 1-4%, or 2-3% or about 3% by weight.

The composition can be further comprising a whitening agent in addition to the ascorbic acid derivative as defined

in the claims.

The oral care composition can be in the form selected from: dentifrice (e.g., a toothpaste or oral gel), powder (e.g., tooth powder), cream, mouthwash, strip or gum (e.g., chewing gum).

The pH of the composition can be from 6 to 9, such as from 6.5 to 8, or from 6.5 to 7.5, or about 7.0.

The composition can be a single-phase composition (e.g., not a dual-phase composition).

The composition can be essentially free or free of phosphates of more than four phosphate groups.

The composition can be essentially free or free of phosphates of more than three phosphate groups.

The composition can be essentially free or free of hexametaphosphate salts (e.g., sodium hexametaphosphate).

The composition can be effective upon application to the oral cavity, e.g., by rinsing, optionally in conjunction with brushing, to (i) reduce or inhibit formation of dental caries, (ii) reduce, repair or inhibit pre-carious lesions of the enamel, e.g., as detected by quantitative light-induced fluorescence (QLF) or electrical caries measurement (ECM), (iii) reduce or inhibit demineralization and promote remineralization of the teeth, (iv) reduce hypersensitivity of the teeth, (v) reduce or inhibit gingivitis, (vi) promote healing of sores or cuts in the mouth, (vii) reduce levels of acid producing bacteria, (viii) to increase relative levels of arginolytic bacteria, (ix) inhibit microbial biofilm formation in the oral cavity, (x) raise and/or maintain plaque pH at levels of at least pH 5.5 following sugar challenge, (xi) reduce plaque accumulation, (xii) treat, relieve or reduce dry mouth, (xiii) clean the teeth and oral cavity (xiv) reduce erosion, (xv) prevents stains and/or whiten teeth, (xvi) immunize the teeth against cariogenic bacteria; and/or (xvii) promote systemic health, including cardiovascular health, e.g., by reducing potential for systemic infection via the oral tissues.

The composition can further comprise a polymer selected from the group consisting of: carboxymethyl cellulose (free form or a salt, e.g., sodium salt), a gum (e.g., xanthan gum, carrageenan gum, or gum arabic), polyethylene glycol (e.g., polyethylene glycol 200, 400, 600 or 800, or a mixture thereof), and a combinations thereof, for example, a mixture of sodium carboxy methyl cellulose, xanthan gum, polyethylene glycol 600.

The polymer can comprise sodium carboxy methyl cellulose.

The polymer can comprise xanthan gum.

The composition can further be comprising a silica thickener and/or a silica abrasive. The oral care composition can comprise an additional anionic surfactant, wherein the anionic surfactant is selected from the group consisting of: water-soluble salts of higher fatty acid monoglyceride monosulfates (such as the sodium salt of the monosulfated monoglyceride of hydrogenated coconut oil fatty acids such as sodium N-methyl N-cocoyl taurate), sodium cocomonoglyceride sulfate, higher alkyl-ether sulfates (e.g., of formula $CH_3(CH_2)_mCH_2(OCH_2CH_2)_nOSO_3X$, wherein m is 6-16, e.g., 10, n is 1-6, e.g., 2, 3 or 4, and X is Na or K, for example sodium laureth-2 sulfate ($CH_3(CH_2)_{10}CH_2(OCH_2CH_2)_2O$-$SO_3Na$)), higher alkyl aryl sulfonates such as sodium dodecyl benzene sulfonate (sodium lauryl benzene sulfonate), higher alkyl sulfoacetates (such as sodium lauryl sulfoacetate (dodecyl sodium sulfoacetate)), higher fatty acid esters of 1,2 dihydroxy propane sulfonate, sulfocolaurate (N-2-ethyl laurate potassium sulfoacetamide), sodium lauryl sulfate, sodium stearate and sodium lauryl sarcosinate.

The composition can comprise an amino acid.

The amino acid can be a basic amino acid (e.g., arginine)
The amino acid can be a basic amino acid provided in the form of a di- or tri-peptide comprising arginine or lysine, or salts thereof.

The basic amino acid can comprise arginine or lysine, and wherein the arginine or lysine is present in an amount corresponding to 1% to 15%, e.g., 3 wt. % to 10 wt. % of the total composition weight, about e.g., 1.5%, 4%, 5%, or 8%, wherein the weight of the basic amino acid is calculated as free form.

The amino acid can comprise arginine from 0.1 wt. % - 6.0 wt. % (e.g., about 1.5 wt.%) (e.g., about 5 wt.%) of the total composition, wherein the weight of the arginine is calculated as free form.

The amino acid can be arginine from about 1.5 wt. of the total composition, wherein the weight of the arginine is calculated as free form.

The amino acid can be arginine from 4.5 wt. % - 8.5 wt. % (e.g., about 5.0 wt.%) of the total composition, wherein the weight of the basic amino acid is calculated as free form.

The amino acid can be arginine from about 5.0 wt. % of the total composition, wherein the weight of the basic amino acid is calculated as free form.

The amino acid can be L-arginine.

The amino acid can be a free form arginine.

The amino acid can be arginine or lysine in partially or wholly in salt form.

The amino acid can be arginine phosphate, arginine hydrochloride or arginine bicarbonate.

The composition is comprising a zwitterionic surfactant as defined in the claims. The zwitterionic surfactant can be a betaine zwitterionic surfactant (e.g., from 0.1% - 5% by wt. of the total composition) (e.g., 0.2% - 1% by wt. of the total composition) (e.g., about 0.6% by wt. of the total composition).

The betaine zwitterionic surfactant can be a C8-C16 aminopropyl betaine (e.g., cocamidopropyl betaine).

The C8-C16 aminopropyl betaine can be cocamidopropyl betaine.

The cocamidopropyl betaine can be present in an amount of from 0.5% to 4% by wt. of the total composition.

The cocamidopropyl betaine can be present in an amount of from 0.1% to 3% by wt. of the total composition.

The cocamidopropyl betaine can be present in an amount of from 0.1% to 1% (e.g., about 0.6% by wt. of the total composition).

The composition can comprise cocamidopropyl betaine and sodium methyl cocoyl taurate in a wt.% ratio of (e.g., wt.%) is from 0.1:1 to 1:1 (e.g., 0.1:1, 0.2:1, 0.3:1, 0.4:1 or 0.5:1) (e.g., 0.3:1).

The oral care composition can be free of sodium lauryl sulfate.

The composition can be further comprising cocamidopropyl betaine, in an amount of from 0.1% to 5% by wt. of the total composition.

Optionally, the composition does not contain any sodium lauryl sulfate.

The composition can be substantially free of sodium lauryl sulfate.

The oral care composition can be a dentifrice (e.g., a toothpaste or oral gel), powder (e.g., tooth powder), cream, mouthwash, strip or gum (e.g., chewing gum).

The compositions can be further comprising a preservative selected from: benzyl alcohol, Methylisothizolinone ("MIT"), Sodium bicarbonate, lauryl alcohol, and polyphosphate.

The compositions can be comprising nitric acid or a water-soluble nitrate salt (e.g., potassium nitrate).

The water-soluble nitrate salt can be selected from an alkali or alkaline earth metal nitrate, or zinc nitrate, silver nitrate, or ammonium nitrate.

The water-soluble nitrate salt can be an alkali metal nitrate salt or an alkaline earth metal nitrate salt.

The nitrate salt can be selected from lithium nitrate, sodium nitrate, potassium nitrate, magnesium nitrate, and calcium nitrate.

The nitrate salt can be potassium nitrate.

The oral care composition can be free or substantially free of sodium lauryl sulfate The oral care composition can comprise an effective amount of a taurate surfactant, wherein the taurate surfactant is represented by Formula (1):

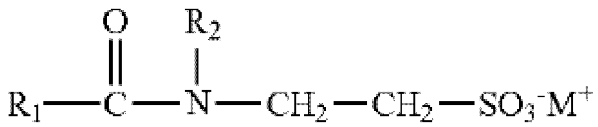

(1)

wherein $R_1$ is a saturated or unsaturated, straight or branched alkyl chain with 6 to 18 C atoms $R_2$ is H or methyl, and $M^+$ is H, sodium, or potassium (e.g., sodium methyl cocoyl taurate).

The $R_1$ can be a saturated or unsaturated, straight or branched alkyl chain with 8 to 14 C atoms.

The taurate surfactant can comprise one or more surfactant selected from the group consisting of: potassium cocoyl taurate, potassium methyl cocoyl taurate, sodium caproyl methyl taurate, sodium cocoyl taurate, sodium lauroyl taurate, sodium methyl cocoyl taurate (SMCT), sodium methyl lauroyl taurate, sodium methyl myristoyl taurate, sodium methyl oleoyl taurate, sodium methyl palmitoyl taurate, sodium methyl stearoyl taurate, and combinations thereof.

The taurate surfactant can comprise sodium methyl cocoyl taurate (e.g., 1% - 5% by wt. of sodium methyl cocoyl taurate) (e.g., about 2% by wt. sodium methyl cocoyl taurate).

The oral care composition can comprise sodium stearate (e.g., from 1% - 5% by wt. of the composition) (e.g., from 1.5% - 3% by wt.) (e.g., from 1.75% - 2.5% by wt.) (e.g., from 1.75% - 2.25% by wt.) (e.g., about 2% by wt.) (e.g., from 2% - 5% by wt.) (e.g., from 2.5% - 4.5% by wt.) (e.g., from 2.75% - 4.25% by wt.) (e.g., from 3% - 4.25% by wt.) (e.g., about 4% by wt.).

The oral care composition can be free from other whitening agents comprising peroxides, percarbonates, peroxysilicates, persulfate derivatives and/or combinations thereof.

The oral care composition can be in the form selected from: toothpaste, dentifrice, mouthwash, mouthrinse, topical oral gel, denture cleanser, dental strips, beads, ribbon toothpaste, varnish, dental floss, tablets, chewing gum, lozenges and toothpowder.

The oral care composition can comprise sodium stearate from 0.1 - 4% by weight relative to the total composition.

The amount of sodium stearate can be from 0.4% - 1.6% by nesosilicates weight relative to the total composition.

The amount sodium stearate can be from 0.5% - 1.5% by nesosilicates weight relative to the total composition.

The amount sodium stearate can be about 0.5% by wt. about 0.75% by wt., about 1% by wt., about 1.25% by wt., or about 1.5% by weight relative to the total composition.

The disclosure also provides a method for whitening a surface of a tooth as defined in the claims.

The patient in need thereof can have gum irritation or is at risk of gum irritation. The patient in need thereof can have weak dental enamel and/or is a risk for weak dental enamel.

The patient in need thereof can suffer from one or more symptoms a gastric disorder.

The one or more symptoms of a gastric disorder can be dental erosion (e.g., tooth enamel erosion) that is consequent to the presence of gastric acid (e.g., stomach acid) in the oral cavity (e.g., increased or elevated amounts of gastric acid).

The dental erosion (e.g., erosion of the tooth enamel) that is consequent to the presence of gastric acid can be the erosion of the subject's tooth enamel from loss of calcium.

The patient can have a gastric disorder that increases the amount of gastric acid in the oral cavity of the patient (and/or prolongs the oral cavity to exposure of gastric acid), and wherein the gastric disorder is selected from the group consisting of: duodenal ulcers, gastric ulcers, gastroesophageal reflux disease (GERD), erosive esophagitis, gastroesophageal reflux disease weakly reactive (poorly responsive symptomatic gastroesophageal reflux disease), bulimia nervosa, pathological gastrointestinal hypersecretory disease (pathological gastrointestinal hypersecretory disease), Zhuo - Ellison syndrome, heartburn, and acid indigestion.

The gastric disorder can be gastroesophageal reflux disease (GERD).

The patient can be at risk for dental erosion.

The patient can be at risk for gingivitis.

The disclosure also provides a method for increasing the color stability of an oral care composition comprising an ascorbic acid derivative as defined in the claims.

The composition can be administered as part of a method to treat or reduce staining of the enamel.

The composition can be used in a method to whiten the enamel surface in order to remove extrinsic staining.

As used herein, "staining" refers to a discoloration of a dental surface caused by adsorption or absorption of a color agent on or into the surface, or caused by reaction of material of the dental surface (e.g., dental enamel) with a color or noncolor agent contacting the surface. In one aspect, that staining is consequent to exposure to tobacco smoke, chewing tobacco, tea and/or coffee.

**Methods of manufacture**

The oral care compositions of the disclosure are made by any variety of methods including adding and mixing the ingredients of the oral care composition in a suitable vessel provided with a mixer. In one embodiment, the ascorbic acid derivative, humectant(s), surfactant(s) and carrier(s) are mixed to form a homogenous mixture. Additional ingredients such as flavoring agents, coloring or sweeteners are added at any point during the mixing process but in various embodiments such ingredients are preferably added last or close to last.

**EXAMPLES**

The following examples are for illustrative purposes only.

As will be evident to those skilled in the art, individual isomeric forms can be obtained by separation of mixtures thereof in conventional manner. For example, in the case of diasteroisomeric isomers, chromatographic separation may be employed.

The following compounds are used in the examples:

| Compound name | CAS # | Chemical structure |
|---|---|---|
| L-(+) ascorbic acid | 50-81-7 | 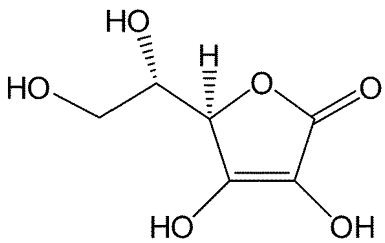 |
| (+) sodium-L-ascorbate | 134-03-2 | 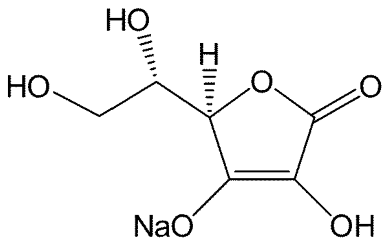 |

(continued)

| Compound name | CAS # | Chemical structure |
|---|---|---|
| sodium ascorbyl phosphate | 66170-10-3 | 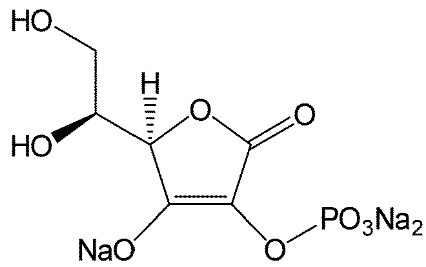 |
| tris(2-carboxyethyl) phosphine hydrochloride (TCEP) | 51805-45-9 | 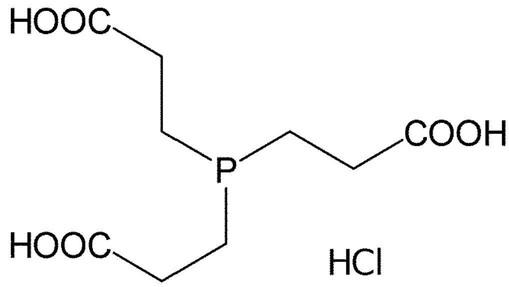 |
| citric acid | 77-92-9 | 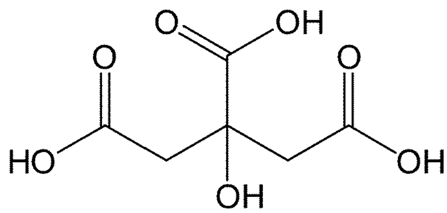 |
| ascorbyl 6-palmitate | 137-66-6 | 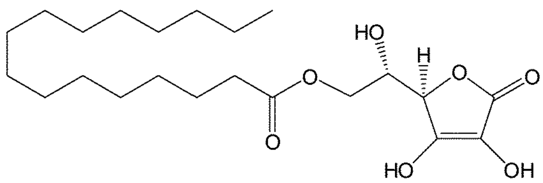 |

Several basic toothpastes comprising a variety of backbones were formulated as described in the following tables. In order to evaluate the whitening efficacy of reducing agents, an array of reducing agents is added to the basic toothpastes in different percentages by weight. The pH of the toothpaste samples is adjusted as needed using 1M sodium hydroxide (NaOH) and 1 M hydrochloric acid (HCl) to maintain a pH between 5 -11. The whitening efficacy of these toothpaste samples is determined in a standard brushing study, as described below in **Example 1.** In each study, the whitening efficacy was compared to the basic toothpaste and to whitening toothpastes comprising more traditional whitening agents such as peroxides and/or abrasives.

**Example 1.** Standard brushing study

Bovine enamel teeth mounted individually in resin blocks are purchased and brushed with an abrasive paste until initial "L" values were 56-68. Trays are prepared for the brushing machine and each tray consists of six teeth mounted using 3D printed trays. Baseline optical measurements of the bovine teeth are taken using a spectrophotometer and the trays are loaded onto the brushing machine.

Toothbrushes are placed on the brushing machine by removing the head of a manual toothbrush from its handle. Slurry composed of 1:1 ratio by weight of test toothpaste to artificial saliva is prepared and added to each tray. The teeth are brushed for two minutes with 250 grams of pressure at 120 strokes per minute. After brushing is stopped, the slurry is removed and residual toothpaste is rinsed away with deionized water. The brushing treatment is repeated a total of 14 times and measurements are taken every 2 cycles. The teeth are then gently blotted with a paper towel to remove excess liquid and measurements are taken with the spectrophotometer. Six teeth are tested in each cell. The "L", "a", and "b" values are determined and are used to calculate the change in whiteness "ΔW" for each tooth after the treatment. The "L",

"a", and "b" values, according to the International Committee of Illumination (CIE), provide a numerical representation of three-dimensional color space where "L" represents a lightness axis, "a" represents a red-green axis and "b" represents a yellow-blue axis. All "ΔW" values are averaged for teeth within the same treatment. A lower "ΔW" means the whiter the teeth.

$$W^* = \sqrt{a^2 + (b^*)^2 + (L^* - 100)^2}$$

$$\Delta W^* = W^*_{treated} - W^*_{baseline}$$

Anova-Tukey statistical analysis is performed to check for statistical difference from buffer alone ($p < 0.05$ declared as significant).

**Example** 2. Evaluation of reducing agents as whitening agents in toothpaste

Basic Toothpaste A is prepared without a coloring agent according to **Table 1.**

**Table** 1: Toothpaste A

| | **INGREDIENT** | **WEIGHT (%)** |
|---|---|---|
| 1 | Humectants<br>(e.g. non-crystal sorbitol, 99.5% vegetable refined glycerin polyhydric alcohols, polyoxyethylene glycols) | 60.06 |
| 2 | Abrasives<br>(e.g. synthetic high cleaning silica, perlite, potassium silicate, synthetic amorphous silica, synthetic abrasive silica, core shell silica, precipitated silica, calcium carbonate, sodium bicarbonate, dicalcium phosphate) | 20.34 |
| 3 | Anionic Surfactants<br>(e.g. sodium carboxymethyl cellulose, polyanionic cellulose, cocoyl monoglyceride sulfonate, sodium lauryl sarcosinate, sodium lauryl isoethionate, sodium laureth carboxylate, sodium lauryl sulfate, sodium cocoyl glutamate, sulfonated monoglycerides of $C_{8-20}$ fatty acids, sodium stearate, lauryl glucoside) | 0.67 |
| 4 | Amphoteric surfactants<br>(e.g. cocamidopropyl betaine, alkyl betaines and alkyl amido betaines) | 1.26 |
| 5 | Thickening Agents<br>(e.g. thickening silica, xanthan gum, gums, carbomers, carrageenans, sodium carboxymethyl cellulose, polyanionic cellulose, fumed silica) | 2.79 |
| 6 | Foam Modulators<br>(e.g. sodium lauroyl sarcosinate, sodium lauryl sulfate powder, polyethylene glycols, sodium lauryl sulfate liquid) | 1.52 |
| 7 | Stain Prevention Agents<br>(e.g. tetrasodium pyrophosphate, disodium pyrophosphate, sodium tripyrophosphate, sodium hexametaphosphate, zinc citrate) | 0.52 |
| 8 | Erosion prevention agents<br>(e.g. Sodium fluoride, arginine, arginine phosphate, arginine hydrochloride, arginine bicarbonate, sodium monofluorophosphate, potassium citrate, titanium phosphate, zinc oxide, zinc citrate, zinc citrate trihydrate, zinc lysine chloride complexes, zinc arginine chloride complexes) | 0.25 |
| 9 | Sweeteners<br>(e.g. saccharin, sucralose, stevia derivatives) | 0.3 |
| 10 | Deionized water | q.s. |

Various reducing agents are added to Toothpaste A in an amount of 2.5% by weight, based on the total weight of the toothpaste sample and the pH is adjusted as necessary.

**Table 2:** Toothpaste A samples comprising reducing agents

| Sample | Reducing agent | Amount of reducing agent added (%) weight |
|---|---|---|
| Toothpaste A (negative control) | none | - |
| 1 | L-ascorbic acid | 2.5 |
| 2 | (+)-sodium L-ascorbate | 2.5 |
| 3 | sodium ascorbyl phosphate | 2.5 |
| 4 | ascorbyl 6-palmitate | 2.5 |
| 5 | citric acid | 2.5 |
| 6 | citric acid + | 2.5 |
| | L-ascorbic acid | 2.5 |
| 7 | TCEP | 2.5 |

The whitening efficacy of Toothpaste A samples is determined in the standard brushing study, as described in **Example 1.** The ΔW values are tabulated in **Table 3.**

**Table 3:** ΔW values for Toothpaste A samples comprising reducing agents

| | **CONTROL** | **SAMPLES** | | | | | | |
|---|---|---|---|---|---|---|---|---|
| **Cycl e** | **TP Aa** | **1[b]** | **2[c]** | **3[d]** | **4[e]** | **5[f]** | **6[g]** | **7[h]** |
| 2 | -1.20 | - 1.23 | -1.27 | -1.49 | -2.97 | -2.02 | -1.59 | -1.20 |
| 4 | -2.30 | - 1.66 | -2.22 | -2.90 | -4.55 | -2.64 | -2.77 | -2.90 |
| 6 | -2.98 | - 2.09 | -2.55 | -3.98 | -6.00 | -4.21 | -3.50 | -3.68 |
| 8 | -4.14 | - 2.65 | -3.26 | -4.64 | -7.13 | -4.23 | -4.15 | -4.26 |
| 10 | -4.53 | - 2.70 | -3.81 | -5.48 | -7.85 | -5.34 | -4.98 | -4.49 |
| 12 | -5.04 | - 3.16 | -4.20 | -6.37 | -8.05 | -5.06 | -5.23 | -5.66 |
| 14 | -5.54 | - 3.47 | -5.13 | -6.49 | -8.32 | -5.45 | -6.20 | -6.25 |

"[a]" TP A is toothpaste A as defined in **Table 1**
"[b]" Toothpaste A comprising 2.5% weight L-ascorbic acid
"[c]" Toothpaste A comprising 2.5% weight (+)-sodium L-ascorbate
"[d]" Toothpaste A comprising 2.5% weight sodium ascorbyl phosphate
"[e]" Toothpaste A comprising 2.5% weight ascorbyl 6-palmitate
"[f]" Toothpaste A comprising 2.5% weight citric acid
"[g]" Toothpaste A comprising 2.5% weight citric acid + 2.5% weight L-ascorbic acid
"[h]" Toothpaste A comprising 2.5% weight TCEP

The ΔW data presented in **Table** 3 show that ascorbic acid salts: sodium ascorbyl phosphate and ascorbyl 6-palmitate provide whitening efficacy when added to Toothpaste A: sample 3 and sample 4, respectively. Based on the results, the whitening efficacy of ascorbyl 6-palmitate is further investigated in a different basic toothpaste formulation.

**Example 3.** Evaluation of ascorbyl 6-palmitate as whitening agent

Basic Toothpaste B is prepared without flavor or coloring agent according to **Table 4.**

**Table 4:** Toothpaste B

| | INGREDIENTS | WEIGHT (%) |
|---|---|---|
| 1 | Humectants<br>(e.g. non-crystal sorbitol, 99.5% vegetable refined glycerin polyhydric alcohols, polyoxyethylene glycols) | 44.6 |
| 2 | Abrasives<br>(e.g., synthetic high cleaning silica, perlite, potassium silicate, synthetic amorphous silica, synthetic abrasive silica, core shell silica, precipitated silica, calcium carbonate, sodium bicarbonate, dicalcium phosphate) | 20.0 |
| 3 | Anionic Surfactants<br>(e.g. sodium carboxymethyl cellulose, polyanionic cellulose, cocoyl monoglyceride sulfonate, sodium lauryl sarcosinate, sodium lauryl, sodium cocoyl glutamate, sodium lauryl isoethionate, sodium laureth carboxylate, sodium lauryl sulfate, sulfonated monoglycerides of $C_{8-20}$ fatty acids, sodium stearate, lauryl glucoside) | 4.4 |
| 4 | Amphoteric surfactants<br>(e.g. cocamidopropyl betaine, alkyl betaines and alkyl amido betaines) | 2.5 |
| 5 | Thickening Agents<br>(e.g. thickening silica, xanthan gum, gums, carbomers, carrageenans, sodium carboxymethyl cellulose, polyanionic cellulose, fumed silica) | 7.8 |
| 6 | Preservatives<br>(e.g., natural benzyl alcohol, chlorhexidine, benzoic acid, benzoic acid salts, quaternary ammonium compounds, benzalkonium chloride) | 0.5 |
| 7 | Erosion prevention agents<br>(e.g., Sodium fluoride, arginine, arginine phosphate, arginine hydrochloride, arginine bicarbonate, sodium monofluorophosphate, potassium citrate, titanium phosphate, zinc oxide, zinc citrate, zinc citrate trihydrate, zinc lysine chloride complexes, zinc arginine chloride complexes) | 0.2 |
| 8 | Demineralized Water | q.s. |

Ascorbyl 6-palmitate is added in different amounts to basic Toothpaste B and the pH of the samples is adjusted as necessary.

**Table 5:** Toothpaste B samples comprising ascorbyl 6-palmitate

| Sample | Whitening agent | Amount of whitening agent added (%) weight |
|---|---|---|
| Toothpaste B (negative control) | none | - |
| 1* | ascorbyl 6-palmitate | 1 |
| 2* | ascorbyl 6-palmitate | 3 |
| 3* | ascorbyl 6-palmitate | 5 |
| *: comparative | | |

The whitening efficacy of Toothpaste B samples is determined in the standard brushing study, as described in **Example** 1. Besides basic Toothpaste B, two more toothpaste samples are used as controls: Toothpaste C and Toothpaste D. Toothpaste C, contains dicalcium phosphate dihydrate and Toothpaste D contains 0.1 % hydrogen peroxide as whitening agent. The ΔW values are tabulated in **Table 6.**

**Table** 6: ΔW values for Comparative Toothpaste B samples comprising ascorbyl 6-palmitate

| Cycle | CONTROLS | | | SAMPLES | | |
|---|---|---|---|---|---|---|
| | **TP B[a]** | **TP C[b]** | **TP D[c]** | **1[d]** | **2[e]** | **3[f]** |
| 2 | -0.86 | -0.78 | -2.41 | -1.99 | -1.96 | -1.88 |
| 4 | -1.13 | -1.42 | -3.91 | -2.76 | -3.02 | -3.79 |
| 6 | -1.69 | -1.43 | -5.12 | -3.55 | -3.95 | -4.72 |
| 8 | -2.26 | -1.69 | -5.77 | -3.95 | -4.31 | -6.47 |
| 10 | -2.77 | -1.96 | -6.21 | -4.47 | -5.09 | -7.06 |
| 12 | -3.15 | -2.35 | -6.36 | -4.78 | -5.68 | -7.15 |
| 14 | -3.73 | -2.26 | -6.90 | -5.32 | -5.98 | -7.36 |

"a" TP B is toothpaste B as defined in **Table 4**
"b" TP C is toothpaste comprising dicalcium phosphate dihydrate
"c" TP D is toothpaste comprising 0.1% weight of $H_2O_2$
"a" Toothpaste B comprising 1.0% weight ascorbyl 6-palmitate
"e" Toothpaste B comprising 3.0% weight ascorbyl 6-palmitate
"f" Toothpaste B comprising 5.0% weight ascorbyl 6-palmitate

The ΔW data in **Table** 6 demonstrate that all the toothpaste samples, containing ascorbyl 6-palmitate, had an impact on whitening efficacy. The formulas of Toothpaste "C" and Toothpaste "D" are as follows:

Table 6a -Toothpaste C

| | INGREDIENTS | **WEIGHT** (%) |
|---|---|---|
| 1 | Humectants<br>(e.g. non-crystal sorbitol, 99.5% vegetable refined glycerin polyhydric alcohols, polyoxyethylene glycols) | 22 |
| 2 | Dicalcium Phosphate Dihydrate | 48 |
| 3 | Sodium Lauryl Sulfate | 1.9 |
| 4 | Amphoteric surfactants<br>(e.g. cocamidopropyl betaine, alkyl betaines and alkyl amido betaines) | 2.5 |
| 5 | Thickening Agents | 1.8 |
| 6 | Polyethylene glycol | 1 |
| 7 | Sodium Fluoride | 0.1 |
| 8 | Sodium monofluorophosphate | 0.76 |
| 9 | Demineralized Water | q.s. |
| 10 | Flavor and Sweetener | 1.3 |
| 11 | Alkali phosphate salt (e.g., tetrasodium pyrophosphate) | 0.25 |

Table 6b -Toothpaste D

| | **INGREDIENTS** | **WEIGHT (%)** |
|---|---|---|
| 1 | Humectants<br>(e.g. non-crystal sorbitol, 99.5% vegetable refined glycerin polyhydric alcohols, polyoxyethylene glycols) | 27 |
| 2 | Propylene glycol | 23 |
| 3 | Anionic surfactant | 2 |
| 4 | Polyethylene glycol | 6.3 |

(continued)

| | INGREDIENTS | WEIGHT (%) |
|---|---|---|
| 5 | Thickening Agents | 1.8 |
| 6 | Abrasive | 20 |
| 7 | Sodium monofluorophosphate | 1.1 |
| 8 | Cross-linked PVP complexed with hydrogen peroxide | 0.25 - 1.0 |
| 9 | Flavor and Sweetener | 1.4 |
| 10 | Alkali phosphate salt (e.g., tetrasodium pyrophosphate) | 2 |
| 11 | Cross-linked polyvinyl pyrrolidone | 6 |
| 12 | Polymer | 7.5 |
| 13 | pH adjustment agent | 0.2 |
| 14 | Thickener | 1.9 |
| 15 | Antioxidant | 0.03 |

**Example 4.** Evaluation of sodium ascorbyl phosphate as whitening agent

Based on the results obtained in **Table** 3, the whitening efficacy of sodium ascorbyl phosphate are further investigated. Toothpaste samples are prepared by adding different amounts of sodium ascorbyl phosphate to basic Toothpaste B of **Table 4.** The pH values of the samples were adjusted as necessary.

**Table 7:** Toothpaste B samples comprising sodium ascorbyl phosphate

| Sample | Whitening agent | Amount of whitening agent added (%) weight |
|---|---|---|
| Toothpaste B | none | - |
| 1* | sodium ascorbyl phosphate | 0.5 |
| 2* | sodium ascorbyl phosphate | 1 |
| 3 | sodium ascorbyl phosphate | 1.5 |
| 4* | sodium ascorbyl phosphate | 3 |
| 5* | sodium ascorbyl phosphate | 5 |
| *: comparative | | |

The whitening efficacy of Toothpaste B samples was determined in the standard brushing study, as described in **Example 1.** Besides basic Toothpaste B, two more toothpaste samples were used as controls: Toothpaste C and Toothpaste D. The ΔW values are tabulated in **Table 8.**

**Table 8:** ΔW values for Toothpaste B samples comprising sodium ascorbyl phosphate

| | CYCLE | CONTROLS | | | SAMPLES | | | | |
|---|---|---|---|---|---|---|---|---|---|
| | | TP B[a] | TP C[b] | TP D[c] | 1[d] | 2[e] | 3[f] | 4[g] | 5[h] |
| 1 | 2 | -0.86 | -0.78 | -2.41 | -1.74 | -1.67 | -2.46 | -1.58 | -2.48 |
| 2 | 4 | -1.13 | -1.42 | -3.91 | -2.97 | -2.96 | -4.13 | -2.25 | -3.42 |
| 3 | 6 | -1.69 | -1.43 | -5.12 | -4.03 | -4.05 | -5.45 | -2.91 | -4.38 |
| 4 | 8 | -2.26 | -1.69 | -5.77 | -4.68 | -4.81 | -6.73 | -3.29 | -5.18 |
| 5 | 10 | -2.77 | -1.96 | -6.21 | -5.21 | -5.48 | -7.76 | -3.71 | -5.66 |
| 6 | 12 | -3.15 | -2.35 | -6.36 | -5.70 | -6.12 | -8.70 | -4.15 | -6.14 |

(continued)

| | CYCLE | CONTROLS | | | SAMPLES | | | | |
|---|---|---|---|---|---|---|---|---|---|
| | | TP B[a] | TP C[b] | TP D[c] | 1[d] | 2[e] | 3[f] | 4[g] | 5[h] |
| 7 | 14 | -3.73 | -2.26 | -6.90 | -6.20 | -6.46 | -9.40 | -4.50 | -6.45 |

"a" TP B is toothpaste B as defined in **Table 4**
"b" TP C is toothpaste comprising dicalcium phosphate dihydrate
"c" TP D is toothpaste comprising 0.1% weight of $H_2O_2$
"d" Toothpaste B comprising 0.5% weight sodium ascorbyl phosphate
"e" Toothpaste B comprising 1.0% weight sodium ascorbyl phosphate
"f" Toothpaste B comprising 1.5% weight sodium ascorbyl phosphate
"g" Toothpaste B comprising 3.0% weight sodium ascorbyl phosphate
"h" Toothpaste B comprising 5.0% weight sodium ascorbyl phosphate

The ΔW data in **Table 8** demonstrate that all the toothpaste samples containing sodium ascorbyl phosphate had an impact on whitening efficacy.

Basic Toothpaste B1 is prepared without a coloring agent according to **Table 8A.** 1.5% weight sodium ascorbyl phosphate is added to the Basic Toothpaste B1.

**Table 8A:** Toothpaste B1

| | INGREDIENTS | WEIGHT (%) |
|---|---|---|
| 1 | Humectants<br>(e.g. non-crystal sorbitol, 99.5% vegetable refined glycerin) | 44.6 |
| 2 | Abrasives<br>(e.g. synthetic high cleaning silica, synthetic amorphous silica, synthetic abrasive silica, precipitated silica) | 20.0 |
| 3 | Surfactants<br>(e.g. sodium carboxymethyl cellulose, polyanionic cellulose, cocoyl monoglyceride sulfonate, sodium lauryl sarcosinate, sodium cocoyl glutamate, sodium lauryl isoethionate, sodium laureth carboxylate, sulfonated monoglycerides of Cs $_{-20}$ fatty acids, sodium stearate, lauryl glucoside) | 9.8 |
| 4 | Thickening Agents<br>(e.g. thickening silica, xanthan gum, gums, fumed silica) | 3.8 |
| 5 | Preservatives<br>(e.g. natural benzyl alcohol, benzoic acid, benzoic acid salts, quaternary ammonium compounds) | 0.5 |
| 6 | Erosion prevention agents<br>(e.g. Sodium fluoride) | 0.24 |
| 7 | Demineralized Water | q.s. |

**Example 5.** Further evaluation of sodium ascorbyl phosphate as whitening agent

Based on the results obtained in **Table 3** and **Table 8,** the whitening efficacy of sodium ascorbyl phosphate are further investigated. Basic Toothpaste E are prepared according to **Table 9.**

**Table 9.** Toothpaste E

| | INGREDIENT | WEIGHT (%) |
|---|---|---|
| 1 | Humectants<br>(e.g., non-crystal sorbitol, 99.5% vegetable refined glycerin polyhydric alcohols, polyoxyethylene glycols) | 52.9 |

(continued)

| | INGREDIENT | WEIGHT (%) |
|---|---|---|
| 2 | Abrasives<br>(e.g., perlite, high cleaning silica, potassium silicate, synthetic amorphous silica, abrasive silica, core shell silica, precipitated silica, calcium carbonate, sodium bicarbonate, dicalcium phosphate) | 7.4 |
| 3 | Anionic Surfactants<br>(e.g., sodium carboxymethyl cellulose, polyanionic cellulose, cocoyl monoglyceride sulfonate, sodium lauryl sarcosinate, sodium lauryl isoethionate, sodium laureth carboxylate, sodium lauryl sulfate, sodium cocoyl glutamate, sulfonated monoglycerides of Cs $_{-20}$ fatty acids, Sodium Stearate, lauryl glucoside) | 0.5 |
| 4 | Amphoteric surfactants<br>(e.g., cocamidopropyl betaine, alkyl betaines and alkyl amido betaines) | 1.5 |
| 5 | Thickening Agents<br>(e.g., thickening silica, xanthan gum, carbomers, carrageenans, sodium carboxymethyl cellulose, polyanionic cellulose, microcrystalline cellulose, fumed silica) | 12.6 |
| 6 | Erosion prevention agents<br>(e.g., sodium fluoride, arginine, arginine phosphate, arginine hydrochloride, arginine bicarbonate, sodium monofluorophosphate, potassium citrate, titanium phosphate, zinc oxide, zinc citrate, zinc citrate trihydrate, zinc lysine chloride complexes, zinc arginine chloride complexes) | 0.3 |
| 7 | Foam modulator<br>(e.g., sodium lauroyl sarcosinate, sodium lauryl sulfate powder, polyethylene glycols, sodium lauryl sulfate liquid) | 5.7 |
| 8 | Demineralized Water | q.s. |

Toothpaste samples are prepared by adding different amounts of sodium ascorbyl phosphate to basic Toothpaste E. The pH values of the samples are adjusted as necessary.

**Table 10:** Toothpaste E samples comprising sodium ascorbyl phosphate

| Sample | Whitening agent | Amount of whitening agent added (%) weight |
|---|---|---|
| Toothpaste E | none | - |
| 1* | sodium ascorbyl phosphate | 0.5 |
| 2* | sodium ascorbyl phosphate | 2.75 |
| 3* | sodium ascorbyl phosphate | 5 |
| *: comparative | | |

The whitening efficacy of the toothpaste samples according to **Table 10** is determined in the standard brushing study, as described in **Example 1.** The only change is the starting "L" 59 - 66. The brushing treatment was repeated a total of 14 times and measurements were taken every 5 cycles. Basic Toothpaste E was used as a control. The ΔW values are tabulated in **Table 11.**

**Table 11:** ΔW values for Toothpaste E samples comprising sodium ascorbyl phosphate

| | Cycle | CONTROL | SAMPLES | | |
|---|---|---|---|---|---|
| | | **TP E[a]** | **1[b]** | **3[c]** | **5[d]** |
| 1 | 5 | -0.90 | -2.19 | -4.06 | -2.92 |
| 2 | 10 | -1.80 | -3.41 | -5.62 | -3.96 |

(continued)

| | Cycle | CONTROL | SAMPLES | | |
|---|---|---|---|---|---|
| | | TP E[a] | 1[b] | 3[c] | 5[d] |
| 3 | 15 | -2.45 | -4.01 | -6.31 | -4.70 |

"[a]" TP E is toothpaste E as defined in **Table 9**
"[b]" Toothpaste E comprising 0.5% weight sodium ascorbyl phosphate
"[c]" Toothpaste E comprising 2.75% weight sodium ascorbyl phosphate
"[a]" Toothpaste E comprising 5.0% weight sodium ascorbyl phosphate

The ΔW data in **Table 11** show that all the toothpaste samples, containing sodium ascorbyl phosphate had an impact on whitening efficacy.

**Example 6.** Further evaluation of sodium ascorbyl phosphate as whitening agent

To further study the effect of sodium ascorbyl phosphate on whitening, basic Toothpaste F is prepared according to **Table 12.**

**Table 12.** Toothpaste F

| | INGREDIENT | WEIGHT (%) |
|---|---|---|
| 1 | Humectants<br>(e.g. non-crystal sorbitol, 99.5% vegetable refined glycerin polyhydric alcohols, polyoxyethylene glycols) | 50.50 |
| 2 | Abrasives<br>(e.g. perlite, high cleaning silica, potassium silicate, synthetic amorphous silica, abrasive silica, core shell silica, precipitated silica, calcium carbonate, sodium bicarbonate, dicalcium phosphate) | 7.10 |
| 3 | Anionic Surfactants<br>(e.g. sodium carboxymethyl cellulose, polyanionic cellulose, cocoyl monoglyceride sulfonate, sodium lauryl sarcosinate, sodium lauryl isoethionate, sodium laureth carboxylate, sodium lauryl sulfate, sodium cocoyl glutamate, sulfonated monoglycerides of $C_{8-20}$ fatty acids, Sodium Stearate, lauryl glucoside) | 0.50 |
| 4 | Amphoteric surfactants<br>(e.g. cocamidopropyl betaine, alkyl betaines and alkyl amido betaines) | 1.30 |
| 5 | Thickening Agents<br>(e.g. thickening silica, xanthan gum, gums, carbomers, carrageenans, sodium carboxymethyl cellulose, polyanionic cellulose, microcrystalline cellulose, fumed silica) | 12.00 |
| 6 | Erosion prevention agents<br>(e.g. Sodium fluoride, arginine, arginine phosphate, arginine hydrochloride, arginine bicarbonate, sodium monofluorophosphate, potassium citrate, titanium phosphate, zinc oxide, zinc citrate, zinc citrate trihydrate, zinc lysine chloride complexes, zinc arginine chloride complexes) | 0.30 |
| 7 | Foam modulator<br>(e.g. sodium lauroyl sarcosinate, sodium lauryl sulfate powder, polyethylene glycols, sodium lauryl sulfate liquid) | 5.50 |
| 8 | Antiplaque agents<br>(e.g. calcium pyrophosphate, calcium carbonate, sodium bicarbonate, alumina, dicalcium orthophosphate dihydrate, n-calcium pyrophosphate, tricalcium phosphate, calcium polymetaphosphate, insoluble sodium polymetaphosphate, precipitate calcium carbonate) | 3.0 |
| 9 | Demineralized Water | q.s. |

Toothpaste samples are prepared by adding sodium ascorbyl phosphate to basic Toothpaste F in different amounts. The pH values of the samples were adjusted as necessary, and shown in **Table 13.**

**Table 13:** Toothpaste F samples comprising sodium ascorbyl phosphate

| Sample | Reducing agent | Amount of Reducing agent added (%) weight |
|---|---|---|
| Toothpaste F | none | - |
| 1* | sodium ascorbyl phosphate | 0.5 |
| 2 | sodium ascorbyl phosphate | 1.5 |
| 3* | sodium ascorbyl phosphate | 2.5 |
| 4* | sodium ascorbyl phosphate | 3.5 |
| 5* | sodium ascorbyl phosphate | 5 |
| *: comparative | | |

The brushing study is performed as described previously in **Example 1.** The only change is the starting "L" 57.3 - 66.3, and there are twelve teeth per cell, and the measurements are on cycle 4, 8, and 14 to keep in line with standard brushing studies vs. the factorial study design.

**Table 14:** ΔW values for Toothpaste F samples comprising sodium ascorbyl phosphate

| | **Cycle** | **CONTROL** | **SAMPLES** | | | | |
|---|---|---|---|---|---|---|---|
| | | **TP F[a]** | **1[b]** | **2[c]** | **3[d]** | **4[e]** | **5[f]** |
| 1 | 4 | -0.84 | -0.83 | -1.63 | -1.64 | -2.53 | -1.42 |
| 2 | 8 | -1.78 | -1.86 | -3.19 | -3.51 | -4.41 | -2.70 |
| 3 | 14 | -2.81 | -2.59 | -4.52 | -4.34 | -5.04 | -3.67 |
| "a" TP F is toothpaste F as defined in **Table 12**<br>"b" Toothpaste F comprising 0.5% weight sodium ascorbyl phosphate<br>"c" Toothpaste F comprising 1.5% weight sodium ascorbyl phosphate<br>"d" Toothpaste F comprising 2.5% weight sodium ascorbyl phosphate<br>"e" Toothpaste F comprising 3.5% weight sodium ascorbyl phosphate<br>"f" Toothpaste F comprising 5.0% weight sodium ascorbyl phosphate | | | | | | | |

The ΔW data in **Table 14** show that the dose of sodium ascorbyl phosphate has a significant effect on whitening efficacy.

The addition of sodium ascorbyl phosphate to basic toothpaste formulas yields an observable yellow color in the toothpaste compositions. To overcome this color issue, surfactants are added to the basic toothpaste formulas in combination with sodium ascorbyl phosphate. Table 15 describes one basic toothpaste formula, to which sodium ascorbyl phosphate and surfactants are added. Three surfactants, sodium stearate, sodium cocoyl glutamate and sodium lauryl isoethionate, are screened, alone and in different combinations with/without sodium ascorbyl phosphate. The color of the final toothpaste formulas was determined, as tabulated in Table 16.

**Table 15:** Basic Toothpaste G

| | **INGREDIENTS** | **WEIGHT (%)** |
|---|---|---|
| 1 | Humectants<br>(e.g. non-crystal sorbitol, 99.5% vegetable refined glycerin) | 51.30 |
| 2 | Abrasives<br>(e.g. synthetic high cleaning silica, precipitated silica) | 20.00 |
| 3 | Anionic Surfactants<br>(e.g. sodium carboxymethyl cellulose, lauryl glucoside) | 3.50 |

(continued)

| | INGREDIENTS | WEIGHT (%) |
|---|---|---|
| 3 | Thickening Agents<br>(e.g. thickening silica, fumed silica, xanthan gum, gums) | 5.00 |
| 4 | Preservatives<br>(e.g. natural benzyl alcohol, chlorhexidine, benzoic acid, benzoic acid salts, quaternary ammonium compounds, benzalkonium chloride) | 0.60 |
| 5 | Demineralized Water | q.s. |

Table 16 describes the samples which were tested for color with a spectrophotometer.

Table 16: L* and b* values for Toothpaste G samples comprising sodium ascorbyl phosphate and surfactants

| Sample | Surfactants | | | sodium ascorbyl phosphat e [wt%] | | Color Results | | |
|---|---|---|---|---|---|---|---|---|
| | sodium stearate [wt%] | Plantapon ® a [wt%] | Tauranol ® b [wt%] | | | L* | a* | b* |
| 1 | 0 | 0 | 0 | 0 | | 83.3 | -0.1 | 0.1 |
| 2 | 0 | 0 | 0 | 1.5 | | 80.6 | 1.1 | 15.9 |
| 3 | 2 | 0 | 0 | 0 | | 88.6 | -0.3 | 1.6 |
| 4 | 4 | 0 | 0 | 0 | | 91.5 | -0.7 | 1 |
| 5 | 0 | 0 | 0.5 | 0 | | 86.7 | -0.3 | 1.3 |
| 6 | 0 | 2.5 | 1 | 0 | | 83.8 | -0.6 | 1.5 |
| 7 | 4 | 2.5 | 0 | 0 | | 87.2 | -0.8 | 1.4 |
| 8 | 4 | 1.25 | 1 | 0 | | 90.4 | -0.6 | 1.5 |
| 9 | 4 | 2.5 | 1 | 0 | | 90.5 | -0.7 | 0.9 |
| 10 | 0 | 0 | 1 | 1.5 | | 81.5 | 0.5 | 14 |
| 11 | 0 | 2.5 | 0 | 1.5 | | 82.8 | 0.3 | 11.1 |
| 12 | 0 | 1.25 | 0 | 1.5 | | 82.6 | 1.2 | 15.3 |
| 13 | 0 | 2.5 | 0 | 0.75 | | 83.4 | 0.7 | 11.4 |
| 14 | 4 | 0 | 0 | 1.5 | | 89 | -0.5 | 2.8 |
| 15 | 0 | 2.5 | 1 | 1.5 | | 83.2 | 0 | 9.6 |
| 16 | 4 | 2.5 | 0 | 1.5 | | 89.1 | -0.5 | 2.1 |
| 17 | 4 | 0 | 1 | 0.75 | | 90.1 | -0.9 | 1.7 |
| 18 | 2 | 1.25 | 0.5 | 1.5 | | 90.9 | -0.4 | 0.7 |
| 19 | 4 | 2.5 | 1 | 1.5 | | 90.2 | -0.8 | 2.3 |
| 20 | 2 | 2.5 | 1 | 1.5 | | 89 | -0.3 | 6.1 |
| 21 | 2 | 1.25 | 0.5 | 0.75 | | 88.9 | -0.8 | 3.7 |
| 22 | 4 | 2.5 | 0.5 | 1.5 | | 91.6 | -0.8 | 3.5 |
| "a" Plantapon® is sodium cocoyl glutamate<br>"b" Tauranol® is sodium lauryl isoethionate | | | | | | | | |

Samples of basic toothpaste G with different amounts and combinations of surfactants with/without sodium ascorbyl phosphate are prepared and evaluated for color, as shown in Table 16. The L*, a*, b* values of these samples are tabulated in Table 16. A high "b*" value indicates a more yellow color while a lower value indicates a more blue color. A high

"L*" value indicates a whiter sample compared to a low "L*" value which indicates a darker sample. As can be observed in Table 16 samples without sodium stearate that incorporate sodium ascorbyl phosphate tends to increase the "b*" value, indicating a more yellow color. Samples that incorporate sodium stearate with sodium ascorbyl phosphate tend to have a relative reduction in "b*" value, indicating less yellow color.

## Claims

1. An oral care composition for whitening dental enamel, wherein the oral care composition comprises an effective amount of an ascorbic acid derivative,

    wherein the ascorbic acid derivative is sodium ascorbyl phosphate;
    wherein the ascorbic acid derivative is present in an amount of about 1.5% by wt. of the total composition; and a zwitterionic surfactant.

2. The oral care composition of claim 1, wherein the amount of the ascorbic acid derivative is sufficient to increase ΔW of the enamel by at least 0.3 times greater, or by 0.3 - 4 times greater, relative to a non-peroxide reference standard.

3. The oral care composition of any of the preceding claims comprising an effective amount of a fluoride ion source.

4. The oral care composition of any of the preceding claims, wherein the oral care composition comprises an abrasive.

5. The oral care composition of any of the preceding claims, comprising one or more humectants, selected from sorbitol, glycerol, xylitol and propylene glycol, or combinations thereof.

6. The oral care composition of any of the preceding claims comprising an anionic surfactant.

7. The oral care composition of any of the preceding claims comprising an effective amount of one or more alkali phosphate salts for example orthophosphates, pyrophosphates, tripolyphosphates, tetraphosphates or higher polyphosphates.

8. The oral care composition of any of the preceding claims comprising an amino acid, wherein the amino acid is a basic amino acid.

9. The oral care composition of any of the preceding claims comprising a stannous ion source, optionally, wherein the stannous ion source comprises stannous fluoride.

10. The oral care composition of any of the preceding claims, wherein the composition comprises a zinc ion source and wherein the zinc ion source comprises one or more zinc salt(s) selected from the group consisting of: zinc citrate, zinc oxide, zinc phosphate, zinc lactate, zinc sulfate, zinc silicate, zinc gluconate and combinations thereof.

11. The oral care composition of any of the preceding claims, wherein the composition comprises a taurate surfactant, wherein the taurate surfactant is selected from the group consisting of: potassium cocoyl taurate, potassium methyl cocoyl taurate, sodium caproyl methyl taurate, sodium cocoyl taurate, sodium lauroyl taurate, sodium methyl cocoyl taurate (SMCT), sodium methyl lauroyl taurate, sodium methyl myristoyl taurate, sodium methyl oleoyl taurate, sodium methyl palmitoyl taurate, sodium methyl stearoyl taurate, and combinations thereof.

12. The oral care composition of any of the preceding claims, wherein the composition comprises sodium stearate in an amount from 0.1 - 4% by wt. relative to the weight of the total composition.

13. A non-therapeutic method for whitening a surface of a tooth, wherein the method comprises contacting the surface of the tooth with an oral care composition of any of claims 1-12, in a patient in need thereof, for a duration of time sufficient to whiten the surface of the tooth.

14. A method for increasing the color stability of an oral care composition comprising an ascorbic acid derivative, wherein the method comprises adding a sodium stearate to a composition of any of claims 1-12, wherein the sodium stearate reduces the amount of yellowing in the composition relative to a reference oral care composition that comprises sodium ascorbyl phosphate or ascorbyl 6-palmitate but does not contain sodium stearate.

15. A non-therapeutic method to treat or reduce staining of the enamel comprising administering an oral care composition of any of claims 1-12, wherein the staining is consequent to exposure to tobacco smoke, chewing tobacco, tea and/or coffee.

**Patentansprüche**

1. Mundpflegezusammensetzung zum Aufhellen von Zahnschmelz, wobei die Mundpflegezusammensetzung eine wirksame Menge eines Ascorbinsäurederivats umfasst,

   wobei das Ascorbinsäurederivat Natriumascorbylphosphat ist;
   wobei das Ascorbinsäurederivat in einer Menge von etwa 1,5 %, bezogen auf das Gewicht der Gesamtzusammensetzung, vorhanden ist; und
   ein zwitterionisches oberflächenaktives Mittel.

2. Mundpflegezusammensetzung nach Anspruch 1, wobei die Menge des Ascorbinsäurederivats ausreichend ist, um $\Delta W$ des Zahnschmelzes um mindestens das 0,3-fache oder um das 0,3- bis 4-fache im Vergleich zu einem nichtperoxidischen Referenzstandard zu erhöhen.

3. Mundpflegezusammensetzung nach einem beliebigen der vorhergehenden Ansprüche, die eine wirksame Menge einer Fluoridionenquelle umfasst.

4. Mundpflegezusammensetzung nach einem beliebigen der vorhergehenden Ansprüche, wobei die Mundpflegezusammensetzung ein Abrasiv umfasst.

5. Mundpflegezusammensetzung nach einem beliebigen der vorhergehenden Ansprüche, die ein oder mehrere Feuchthaltemittel umfasst, die ausgewählt sind aus Sorbit, Glycerin, Xylit und Propylenglykol oder Kombinationen davon.

6. Mundpflegezusammensetzung nach einem beliebigen der vorhergehenden Ansprüche, die ein anionisches oberflächenaktives Mittel umfasst.

7. Mundpflegezusammensetzung nach einem beliebigen der vorhergehenden Ansprüche, die eine wirksame Menge eines oder mehrerer Alkaliphosphatsalze umfasst, beispielsweise Orthophosphate, Pyrophosphate, Tripolyphosphate, Tetraphosphate oder höhere Polyphosphate.

8. Mundpflegezusammensetzung nach einem beliebigen der vorhergehenden Ansprüche, die eine Aminosäure umfasst, wobei die Aminosäure eine basische Aminosäure ist.

9. Mundpflegezusammensetzung nach einem beliebigen der vorhergehenden Ansprüche, die eine Zinn(II)-ionenquelle umfasst, wobei die Zinn(II)-ionenquelle gegebenenfalls Zinn(II)-fluorid umfasst.

10. Mundpflegezusammensetzung nach einem beliebigen der vorhergehenden Ansprüche, wobei die Zusammensetzung eine Zinkionenquelle umfasst und wobei die Zinkionenquelle ein oder mehrere Zinksalz(e) umfasst, die ausgewählt sind aus der Gruppe bestehend aus Zinkcitrat, Zinkoxid, Zinkphosphat, Zinklactat, Zinksulfat, Zinksilikat, Zinkgluconat und Kombinationen davon.

11. Mundpflegezusammensetzung nach einem beliebigen der vorhergehenden Ansprüche, wobei die Zusammensetzung ein Taurat-oberflächenaktives Mittel umfasst, wobei das Taurat-oberflächenaktive Mittel ausgewählt ist aus der Gruppe bestehend aus: Kaliumcocoyltaurat, Kaliummethylcocoyltaurat, Natriumcaproylmethyltaurat, Natriumcocoyltaurat, Natriumlauroyltaurat, Natriummethylcocoyltaurat (SMCT), Natriummethyllauroyltaurat, Natriummethylmyristoyltaurat, Natriummethyloleoyltaurat, Natriummethylpalmitoyltaurat, Natriummethylstearoyltaurat und Kombinationen davon.

12. Mundpflegezusammensetzung nach einem beliebigen der vorhergehenden Ansprüche, wobei die Zusammensetzung Natriumstearat in einer Menge von 0,1 bis 4 %, bezogen auf das Gewicht der Gesamtzusammensetzung, umfasst.

**13.** Nicht-therapeutisches Verfahren zum Aufhellen einer Oberfläche eines Zahns, wobei das Verfahren das Inkontaktbringen der Oberfläche eines Zahns mit einer Mundpflegezusammensetzung nach einem beliebigen der Ansprüche 1 bis 12 bei einem Patienten, der dies benötigt, umfasst, für eine Zeitdauer, die ausreichend ist, um die Zahnoberfläche aufzuhellen.

**14.** Verfahren zum Erhöhen der Farbstabilität einer Mundpflegezusammensetzung, die ein Ascorbinsäurederivat umfasst, wobei das Verfahren die Zugabe eines Natriumstearats zu einer Zusammensetzung nach einem beliebigen der Ansprüche 1 bis 12 umfasst, wobei das Natriumstearat das Ausmaß des Vergilbens in der Zusammensetzung im Vergleich zu einer Referenz-Mundpflegezusammensetzung, die Natriumascorbylphosphat oder Ascorbyl-6-palmitat umfasst, aber kein Natriumstearat umfasst, verringert.

**15.** Nicht-therapeutisches Verfahren zur Behandlung oder Verringerung der Verfärbung des Zahnschmelzes, umfassend das Verabreichen einer Mundpflegezusammensetzung nach einem beliebigen der Ansprüche 1 bis 12, wobei die Verfärbung die Folge der Exposition von Tabakrauch, Kautabak, Tee und/oder Kaffee ist.

## Revendications

**1.** Composition de soins bucco-dentaires pour le blanchiment de l'émail dentaire, dans laquelle la composition de soins bucco-dentaires comprend une quantité efficace d'un dérivé d'acide ascorbique,

dans laquelle le dérivé d'acide ascorbique est le phosphate d'ascorbyle de sodium ;
dans laquelle le dérivé d'acide ascorbique est présent en une quantité d'environ 1,5 % en poids de la composition totale ; et
un tensioactif zwitterionique.

**2.** Composition de soins bucco-dentaires selon la revendication 1, dans laquelle la quantité du dérivé d'acide ascorbique est suffisante pour augmenter le $\Delta W$ de l'émail d'au moins 0,3 fois plus, ou de 0,3 à 4 fois plus, par rapport à un étalon de référence non peroxyde.

**3.** Composition de soins bucco-dentaires selon l'une quelconque des revendications précédentes comprenant une quantité efficace d'une source d'ions fluorure.

**4.** Composition de soins bucco-dentaires selon l'une quelconque des revendications précédentes, dans laquelle la composition de soins bucco-dentaires comprend un abrasif.

**5.** Composition de soins bucco-dentaires selon l'une quelconque des revendications précédentes, comprenant un ou plusieurs humectants, choisis parmi le sorbitol, le glycérol, le xylitol et le propylène glycol, ou les combinaisons de ceux-ci.

**6.** Composition de soins bucco-dentaires selon l'une quelconque des revendications précédentes comprenant un tensioactif anionique.

**7.** Composition de soins bucco-dentaires selon l'une quelconque des revendications précédentes comprenant une quantité efficace d'un ou plusieurs sels de phosphate alcalins, par exemple des orthophosphates, des pyrophosphates, des tripolyphosphates, des tétraphosphates ou des polyphosphates supérieurs.

**8.** Composition de soins bucco-dentaires selon l'une quelconque des revendications précédentes comprenant un acide aminé, dans laquelle l'acide aminé est un acide aminé basique.

**9.** Composition de soins bucco-dentaires selon l'une quelconque des revendications précédentes comprenant une source d'ions stanneux, éventuellement, dans laquelle la source d'ions stanneux comprend du fluorure stanneux.

**10.** Composition de soins bucco-dentaires selon l'une quelconque des revendications précédentes, dans laquelle la composition comprend une source d'ions zinc et dans laquelle la source d'ions zinc comprend un ou plusieurs sel(s) de zinc choisi(s) dans le groupe constitué par : le citrate de zinc, l'oxyde de zinc, le phosphate de zinc, le lactate de zinc, le sulfate de zinc, le silicate de zinc, le gluconate de zinc et les combinaisons de ceux-ci.

**11.** Composition de soins bucco-dentaires selon l'une quelconque des revendications précédentes, dans laquelle la composition comprend un tensioactif taurate, dans laquelle le tensioactif taurate est choisi dans le groupe constitué par : le cocoyl taurate de potassium, le méthyl cocoyl taurate de potassium, le caproyl méthyl taurate de sodium, le cocoyl taurate de sodium, le lauroyl taurate de sodium, le méthyl cocoyl taurate de sodium (SMCT), le méthyl lauroyl taurate de sodium, le méthyl myristoyl taurate de sodium, le méthyl oléoyl taurate de sodium, le méthyl palmitoyl taurate de sodium, le méthyl stéaroyl taurate de sodium et les combinaisons de ceux-ci.

**12.** Composition de soins bucco-dentaires selon l'une quelconque des revendications précédentes, dans laquelle la composition comprend du stéarate de sodium en une quantité de 0,1 à 4 % en poids par rapport au poids de la composition totale.

**13.** Procédé non thérapeutique pour le blanchiment d'une surface d'une dent, dans lequel le procédé comprend la mise en contact de la surface de la dent avec une composition de soins bucco-dentaires selon l'une quelconque des revendications 1 à 12, chez un patient en ayant besoin, pendant une durée suffisante pour blanchir la surface de la dent.

**14.** Procédé d'augmentation de la stabilité de couleur d'une composition de soins bucco-dentaires comprenant un dérivé d'acide ascorbique, dans lequel le procédé comprend l'ajout d'un stéarate de sodium à une composition selon l'une quelconque des revendications 1 à 12, dans lequel le stéarate de sodium réduit la quantité de jaunissement dans la composition par rapport à une composition de soins bucco-dentaires de référence qui comprend du phosphate d'ascorbyle de sodium ou du 6-palmitate d'ascorbyle mais ne contient pas de stéarate de sodium.

**15.** Procédé non thérapeutique pour traiter ou réduire la coloration de l'émail comprenant l'administration d'une composition de soins bucco-dentaires selon l'une quelconque des revendications 1 à 12, dans lequel la coloration est consécutive à une exposition à la fumée de tabac, au tabac à chiquer, au thé et/ou au café.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004041228 A1
- US 20080112903 A1
- US 20190185499 A1
- JP 2007308443 A
- US 2004175332 A1
- WO 2010074025 A1